# EUROPEAN PATENT SPECIFICATION

(11) **EP 2 487 195 B1**
(45) Date of publication and mention of the grant of the patent: **08.11.2017**
(21) Application number: 12000826.3
(22) Date of filing: 08.02.2012
(51) Int. Cl.: C08G 77/50, C08L 83/14, A61K 8/89, A61K 8/895, C08K 5/10, C08K 5/5419

(54) **Paste composition and cosmetic containing it**
Pastenzusammensetzung und Kosmetik damit
Composition de pâte et produit cosmétique la contenant

(30) Priority: 09.02.2011 JP 2011026330
(43) Date of publication of application: 15.08.2012
(73) Proprietor: Shin-Etsu Chemical Co., Ltd., Tokyo (JP)
(72) Inventor: Ikeda, Teruki, Annaka-shi Gunma-ken (JP)
(74) Representative: Wibbelmann, Jobst

(56) References cited:
- EP-A2- 0 964 023
- WO-A-2004/052982

## Description

### BACKGROUND OF THE INVENTION

### 1. Field of the Invention

The present invention relates to a paste composition and a cosmetic containing it.

### 2. Description of the Related Art

An oil material such as a silicone oil is used in various fields including cosmetics and foods because of its characteristics, safety, and so on. Especially in the fields of cosmetics for a skin care, a make-up, and so on, a silicone oil is blended thereinto with the aim to improve such effects as protective, softening, and moisturizing effects to a skin, and a use feeling. Especially a low-viscosity silicone oil having the dynamic viscosity of 100 or less mm²/second is widely used, because of its excellent spreading properties and a refreshing feeling; but a silicone oil has been difficult to be blended into a cosmetic stably because of its poor compatibility with other cosmetic oil material and low viscosity.

Accordingly, a proposal was made to blend a cosmetic with a paste composition obtained by mixing a specific silicone polymer with an oil material such as a low-viscosity silicone oil under a shear force (Japanese Patent No. 2582275). However, although the paste composition obtained by this method had an improved blending stability, there was a problem that an oily feeling such as slimy and oil film were felt. In U. S. Patent No. 5654362 and Japanese Patent Application Publication No. 2005-537364, a proposal was made to blend a cosmetic with a paste composition obtained by mixing an oil material such as a silicone oil with an elastomer composition comprising a diluent and a crosslinked elastomer, which is comprised of a diene compound and a polysiloxane having a Si-H group; but, there has been a problem here that the diene compound typified by 1,4-pentadiene and 1,5-hexadiene has an unpleasant peculiar odor, so that the composition thereof is not suitable in a cosmetic use if the diene compound is remained therein.

On the other hand, with the aim to improve use feelings such as smoothness and silky, which are characteristics to an oil material such as a silicone oil, a proposal was made to blend a cosmetic with a paste composition obtained by mixing a spherical silicone particle with the oil material such as a silicone oil. For example, a proposal was made to blend a cosmetic with a composition comprising a spherical crosslinked silicon particle having a specific particle diameter and an oil material such as a silicone oil (Japanese Patent No. 3488573 and Japanese Patent No. 4025454). In the paste composition obtained by the method shown therein, it could be seen the effect that use feelings such as smoothness and silky were improved, but a thickening effect of the spherical silicone particle to the oil material such as a silicone oil was insufficient. Accordingly, in order to be low flowability the paste in consideration of a blending stability thereof to a cosmetic, a large amount of the spherical silicone particle has been necessary for blending; and on top of it, there has been a problem of a storage stability such as separation of the oil material such as a silicone oil and agglomeration of the spherical silicone particles. Consequently, there has not been developed yet a paste composition capable of enhancing a blending stability of the oil material such as a silicone oil to a cosmetic while utilizing characteristics of the oil material such as a silicone oil.

As discussed above, a paste composition capable of enhancing a blending stability of the oil material such as a silicone oil to a cosmetic while giving a silky and light feeling without poor spreading properties and without such feelings as an oily, a sticky, and an oil-film feeling has been wanted to be developed; and in addition, a cosmetic containing this composition has been wanted.

### SUMMARY OF THE INVENTION

The present invention was made in view of the situation as described above, and was intended to provide a paste composition capable of enhancing a blending stability of an oil material such as a silicone oil to a cosmetic while giving a silky and light feeling without poor spreading properties and without such feelings as an oily, a sticky, and an oil-film feeling; and to provide a cosmetic containing this composition.

To solve the problems mentioned above, the present invention provides a paste composition comprising at least:
(I) an organopolysiloxane polymer obtained by an addition polymerization of a polysiloxane mixture containing:
   (i) one or more organohydrogen polysiloxane shown by the following general formula (1) 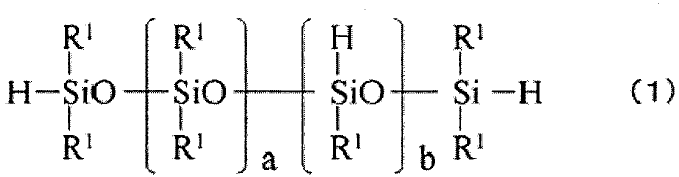 wherein each R¹ may be the same or different and represents a monovalent hydrocarbon group having 1 to 8 carbon atoms and not containing an aliphatic unsaturated group, wherein each of the "a" and "b" represents an integer satisfying a≥1 and b≥0; and
   (ii) one or more organopolysiloxane having an aliphatic unsaturated group and shown by the following general formula (3); 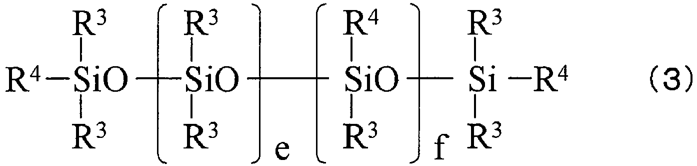 wherein each R³ may be the same or different and represents a monovalent hydrocarbon group having 1 to 8 carbon atoms and not containing an aliphatic unsaturated group, and each R⁴ may be the same or different and represents a monovalent hydrocarbon group having 2 to 8 carbon atoms and containing an aliphatic unsaturated group at its terminal, wherein
      each of the "e" and "f" represents an integer satisfying e≥1 and f≥0, wherein 60% or more by mass relative to the total mass of the polysiloxanes shown by the general formulae (1) and (3) is at least two kinds of the polysiloxanes satisfying any of (a+b)≥30 and (e+f)≥30, with the amounts thereof, and
(II) at least one oil material that is a liquid at 25°C, selected from a silicone oil, a hydrocarbon oil, an ester oil, a natural plant and animal oil, and a semi-synthetic oil.

Further embodiments of the invention are set forth in the claims dependent on claim 1.

In the paste composition as mentioned above, a blending stability of an oil material such as silicon oil to a cosmetic can be made high because the organopolysiloxane polymer (I) has a high thickening effect to the oil material (II) such as a silicone oil; and in addition, the paste composition containing them can give a silky and light feeling without poor spreading properties and without such feelings as an oily, a sticky, and an oil-film feeling.

Further, R⁴ in the general formula (3) is preferably a vinyl group.

The organopolysiloxane polymer (I), obtained by an addition polymerization of the polysiloxane mixture containing one or more organopolysiloxane (ii) as mentioned above, is preferable because it especially shows a high thickening effect to the oil material (II) such as a silicone oil.

In addition, it is preferable that the oil material be contained therein with the mass ratio thereof to the organopolysiloxane polymer being 1/20 to 20/1.

The paste composition containing the organopolysiloxane polymer (I) and the oil material (II) such as a silicone oil with the ratio as described above is preferable because the composition further giving a silky and light feeling without poor spreading properties and without such feelings as an oily, a sticky, and an oil-film feeling can be obtained.

Further, the present invention provides a cosmetic, wherein the paste composition is contained therein.

A cosmetic containing the paste composition as described above can give a silky and light feeling without poor spreading properties and without such feelings as an oily, a sticky, and an oil-film feeling.

As explained above, in the paste composition of the present invention, a blending stability to a cosmetic can be enhanced because the organopolysiloxane polymer (I) has a high thickening effect to the oil material (II) such as a silicone oil; and in addition, the paste composition containing them can give a silky and light feeling without poor spreading properties and without such feelings as an oily, a sticky, and an oil-film feeling. In addition, a cosmetic of the present invention containing the paste composition as described above can give a silky and powdery feeling without poor spreading properties and without such feelings as an oily, a sticky, and an oil-film feeling even if a powder such as a spherical particle is not blended thereinto.

### DETAILED DESCRIPTION OF THE PREFERRED EMBODIMENTS

Hereinafter, a paste composition and a cosmetic containing it, according to the present invention, will be explained in more detail; but the present invention is not limited to these descriptions.

As mentioned above, a paste composition having a high blending stability of an oil material such as a silicone oil to a cosmetic while giving a silky and light feeling without poor spreading properties and without such feelings as an oily, a sticky, and an oil-film feeling has been wanted to be developed; and in addition, a cosmetic containing this composition has been wanted.

As the result of extensive research to solve the problems described above, the present inventors found that, an organopolysiloxane polymer (I) obtained by an addition polymerization of a polysiloxane mixture containing (i) one or more organohydrogen polysiloxane shown by the following general formula (1) or (2) and (ii) one or more organopolysiloxane having an aliphatic unsaturated group and shown by the following general formula (3), wherein 40% or more by mass relative to the total mass of the polysiloxanes shown by the general formulae (1) to (3) is at least two kinds of the polysiloxanes satisfying any of (a+b)≥30, (c+d)≥30, and (e+f)≥30, with the amounts thereof, could show a high thickening effect to an oil material such as a silicone oil; and also found that a paste composition containing this organopolysiloxane polymer and at least one oil material (II) that is a liquid at 25°C, selected from a silicone oil, a hydrocarbon oil, an ester oil, a natural plant and animal oil, and a semi-synthetic oil, could enhance a blending stability of the oil material such as a silicone oil to a cosmetic and give a silky and light feeling without poor spreading properties and without such feelings as an oily, a sticky, and an oil-film feeling. Based on these findings, the present invention could be accomplished.

In addition, the present inventors of the present invention found that a cosmetic giving a silky and light feeling without poor spreading properties and without such feelings as an oily, a sticky, and an oil-film feeling could be obtained if this paste composition was contained therein; and based on this, the present invention could be accomplished. Hereinafter, a detailed explanation will be made.

Namely, in the present invention, provided is a paste composition comprising at least:
(I) an organopolysiloxane polymer obtained by an addition polymerization of a polysiloxane mixture containing:
   (i) one or more organohydrogen polysiloxane shown by the following general formula (1) 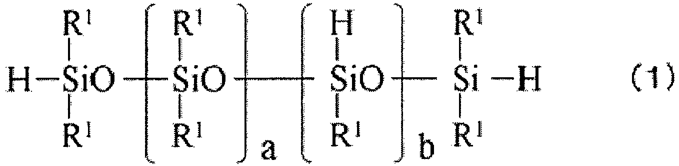 wherein each R¹ may be the same or different and represents a monovalent hydrocarbon group having 1 to 8 carbon atoms and not containing an aliphatic unsaturated group, wherein each of the "a" and "b" represents an integer satisfying a≥1 and b≥0; and (ii) one or more organopolysiloxane having an aliphatic unsaturated group and shown by the following general formula (3); 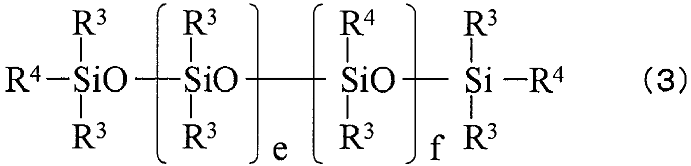 wherein each R³ may be the same or different and represents a monovalent hydrocarbon group having 1 to 8 carbon atoms and not containing an aliphatic unsaturated group, and each R⁴ may be the same or different and represents a monovalent hydrocarbon group having 2 to 8 carbon atoms and containing an aliphatic unsaturated group at its terminal, wherein each of the "e" and "f" represents an integer satisfying e≥1 and f≥0, wherein 60% or more by mass relative to the total mass of the polysiloxanes shown by the general formulae (1) and (3) is at least two kinds of the polysiloxanes satisfying any of (a+b)≥30 and (e+f)≥30, with the amounts thereof, and
(II) at least one oil material that is a liquid at 25°C, selected from a silicone oil, a hydrocarbon oil, an ester oil, a natural plant and animal oil, and a semi-synthetic oil.

The present invention further provides a paste composition as outlined above, wherein the organopolysiloxane polymer obtained by an addition polymerization of a polysiloxane mixture further containing:
one or more organohydrogen polysiloxane shown by the following general formula (2); 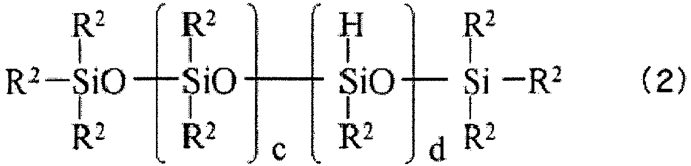 wherein each R² may be the same or different and represents a monovalent hydrocarbon group having 1 to 8 carbon atoms and not containing an aliphatic unsaturated group, wherein each of the "c" and "d" represents an integer satisfying c≥0 and d≥1;
wherein 60% or more by mass relative to the total mass of the polysiloxanes shown by the general formulae (1) to (3) is at least two kinds of the polysiloxanes satisfying any of (a+b) ≥30, (c+d)≥30, and (e+f)≥30, with the amounts thereof.

### [(i) Organohydrogen polysiloxane]

In the general formula (1), each R¹ may be the same or different and represents a monovalent hydrocarbon group having 1 to 8 carbon atoms and not containing an aliphatic unsaturated group, wherein each of the "a" and "b" represents an integer satisfying a≥1 and b≥0. Illustrative example of R¹ includes an alkyl group such as a methyl group, an ethyl group, a propyl group, a butyl group, a pentyl group, a hexyl group, a heptyl group, and an octyl group, as well as a phenyl group, wherein 70% by mol or more relative to the total monovalent hydrocarbon groups represented by R¹ is preferably a methyl group. Although the "a" satisfies a≥1, "a" is preferably 2 to 500, or more preferably 5 to 300, from the viewpoints of a swelling property of the organopolysiloxane polymer (I), obtained by the polymerization, to the oil material (II) and feeling property of the paste composition. As to the "b", which represents a number of the repeating unit to form a crosslinking structure (unit shown by R¹HSiO_{2/2}) by the addition reaction with the organopolysiloxane (ii) having an aliphatic unsaturated group, the number is preferably 1 to 20, or more preferably 1 to 10, because, if the number of the unit to form this crosslinking structure is 20 or less, a swelling property of the organopolysiloxane polymer (I), obtained by the polymerization, to the oil material is increased, while, if the number of the unit is one or more, a sticky feeling of the paste composition is further depressed.

In the general formula (2), each R² may be the same or different and represents a monovalent hydrocarbon group having 1 to 8 carbon atoms and not containing an aliphatic unsaturated group, wherein each of the "c" and "d" represents an integer satisfying c≥0 and d≥1. Illustrative example of R² includes an alkyl group such as a methyl group, an ethyl group, a propyl group, a butyl group, a pentyl group, a hexyl group, a heptyl group, and an octyl group, as well as a phenyl group, wherein 70% by mol or more of the total monovalent hydrocarbon groups represented by R² is preferably a methyl group. Although the "c" satisfies c≥0, "c" is preferably 2 to 300, or more preferably 5 to 200, in view of a swelling property of the organopolysiloxane polymer (I), obtained by the polymerization, to the oil material (II) and feeling property of the paste composition. As to the "d", which represents a number of the repeating unit to form a crosslinking structure (unit shown by R²HSiO_{2/2}) by the addition reaction with the organopolysiloxane (ii) having an aliphatic unsaturated group, the number is preferably 1 to 20, or more preferably 2 to 10, because, if the number of the unit to form this crosslinking structure is 20 or less, a swelling property of the organopolysiloxane polymer (I), obtained by the polymerization, to the oil material is increased, while, if the number of the unit is one or more, a sticky feeling of the paste composition is further depressed.

### [(ii) Organopolysiloxane having an aliphatic unsaturated group]

In the general formula (3), each R³ may be the same or different and represents a monovalent hydrocarbon group having 1 to 8 carbon atoms and not containing an aliphatic unsaturated group. Illustrative example of R³ includes an alkyl group such as a methyl group, an ethyl group, a propyl group, a butyl group, a pentyl group, a hexyl group, a heptyl group, and an octyl group, as well as a phenyl group, wherein 70% by mol or more of the total monovalent hydrocarbon groups represented by R³ is preferably a methyl group. In the general formula (3), each R⁴ may be the same or different and represents a monovalent hydrocarbon group having 2 to 8 carbon atoms and containing an aliphatic unsaturated group at its terminal. Illustrative example of R⁴ includes a vinyl group, an allyl group, a propenyl group, a butenyl group, a pentenyl group, and a hexenyl group, while a vinyl group is preferable. The organopolysiloxane polymer (I) obtained by the addition polymerization of the polysiloxane mixture containing one or more organopolysiloxane (ii) whose R⁴ is a vinyl group in the general formula (3) is preferable, because it shows an especially high thickening effect to the oil material (II) such as a silicone oil. In the general formula (3), each of the "e" and "f" represents an integer satisfying e≥1 and f≥0. Although the "e" satisfies e≥1, "e" is preferably 2 to 500, or more preferably 5 to 300, in view of a swelling property of the organopolysiloxane polymer (I), obtained by the polymerization, to the oil material (II) and feeling property of the paste composition. The "f" is the numbers satisfying f≥0, preferably 10≥f≥0, or more preferably 5≥f≥0.

### [(I) Organopolysiloxane polymer]

The organopolysiloxane polymer (I) is obtained by an addition polymerization of a polysioxane mixture containing (i) one or more organohydrogen polysiloxane shown by the general formula (1) or (2) and (ii) one or more organopolysiloxane having an aliphatic unsaturated group and shown by the general formula (3), wherein the polysiloxane mixture contains at least two kinds of the polysiloxanes satisfying any of (a+b) ≥30, (c+d)≥30, and (e+f)≥30, with the amounts thereof being 40% or more by mass, preferably 60% or more by mass, or more preferably 70% or more by mass, relative to the total mass of the polysiloxanes shown by the general formulae (1) to (3). The addition polymerization is carried out at a temperature of 20 to 150°C in the presence of a catalyst known to those skilled in the art for an addition polymerization, such as for example, a platinum compound such as chloroplatinic acid, an alcohol-modified chloroplatinic acid, and a chloroplatinic acid-vinyl siloxane complex, and a compound of platinum group metal such as rhodium, and palladium. The amount of the catalyst for the addition polymerization may be with an effective amount known to those skilled in the art; and thus, the amount is usually 0.1 to 500 ppm, preferably 0.5 to 200 ppm, or more preferably 1 to 100 ppm, in terms of the converted mass to the platinum group metal.

The addition polymerization may be carried out without a solvent or with an organic solvent as necessary. Illustrative example of the organic solvent includes an alcohol such as methanol, ethanol, 2-propanol, and butanol; an aromatic hydrocarbon such as benzene, toluene, and xylene; an aliphatic or an alicyclic hydrocarbon such as n-pentane, n-hexane, and cyclohexane; a halogenated hydrocarbon such as dichloromethane, chloroform, and carbon tetrachloride; and a ketone such as acetone and methyl ethyl ketone. Further, the addition polymerization may be carried out by the method described in Japanese Examined Patent Publication No. H06-55897, namely, in co-existence of a non-reactive silicone oil or an organic oil. Especially in view of its use as a cosmetic, the reaction is carried out preferably without a solvent, or with a solvent of ethanol or 2- propanol, or in co-existence of a non-reactive silicone oil or an organic oil. Meanwhile, if 40% or more by mass relative to the total mass of the polysiloxanes included in the polysiloxane mixture and shown by the general formulae (1) to (3) is not at least two kinds of the polysiloxanes satisfying any of (a+b)≥30, (c+d)≥30, and (e+f)≥30, with the amounts thereof, the organopolysiloxane polymer obtained even by the addition polymerization thereof cannot give a sufficiently high thickening effect to the oil material such as a silicone oil, thereby leading to a paste composition not giving a powdery, unoily feeling. Further, if (a+b), (c+d), and (e+f) of the each polysiloxanes is less than 30, or if only one polysiloxane satisfies any of (a+b) ≥30, (c+d)≥30, and (e+f)≥30, the paste composition may give a heavy and oily feeling.

The organopolysiloxane polymer (I) of the present invention can be swelled by including thereto at least 100 parts by mass of the oil material (II) relative to 100 parts by mass of the organopolysiloxane polymer. Confirmation of swelling can be made as following. The organopolysiloxane polymer (I) and the oil material (II) that is a liquid at 25°C is kneaded with a three-rod roller; and then visual observation is made to confirm that the oil material (II) that is a liquid at 25°C is not leached out even when the kneaded mixture thereby obtained is allowed to stand on a 100-mesh net for one hour.

### [(II) Oil material]

The oil material (II) contained in the paste composition of the present invention is at least one oil material that is a liquid at 25°C, selected from a silicone oil, a hydrocarbon oil, an ester oil, a natural plant and animal oil, and a semi-synthetic oil. The oil material (II) used in the present invention is an oil material that is a liquid at 25°C, wherein an oil material with the dynamic viscosity at 25°C being 0.65 to 10000 mm²/second is especially preferable. Illustrative example of the oil material (II) includes the following silicone oils, hydrocarbon oils, ester oils, natural plant and animal oils, and semi-synthetic oils.

Illustrative example of the silicone oil includes an organopolysiloxane, having the viscosity of 0.65 to 10000 mm²/second or preferably 0.65 to 1000 mm²/second, such as dimethyl polysiloxane, methyl phenyl polysiloxane, and dimethylsiloxane/methyl phenyl siloxane copolymer; a cyclic siloxane such as octamethyl cyclotetrasiloxane, decamethyl cyclopentasiloxane, and dodecamethyl cyclohexasiloxane; a branched siloxane such as tristrimethyl siloxymethyl silane and tetrakistrimethyl siloxysilane; caprylyl methicone, an alkyl-modified silicone, an amino-modified silicone, and a fluorine-modified silicone.

Illustrative example of the hydrocarbon oil includes a chain or a cyclic hydrocarbon such as an α-olefin oligomer, a light isoparaffin, a light liquid isoparaffin, squalane, a synthetic squalane, a plant squalane, a liquid paraffin, a liquid isoparaffin, a hydrogenated isobutene, isododecane, and isohexadecane.

Illustrative example of the ester oil includes dioctyl succinate, diisobutyl adipate, dioctyl adipate, di(2-heptylundecyl) adipate, diisopropyl sebacate, dioctyl sebacate, dibutyloctyl sebacate, diisostearyl malate, triethyl citrate, ethylene glycol dioctanoate, neopentyl glycol dioctanoate, propylene glycol dicaprate, neopentyl glycol dicaprate, trimethylolpropane trioctanoate, trimethylolpropane triisostearate, pentaerythritol tetraoleate, ethyl acetate, butyl acetate, amyl acetate, octyldodecyl neopentanoate, cetyl octanoate, isononyl isononanoate, isotridecyl isononanoate, hexyldecyl dimethyloctanoate, ethyl laurate, hexyl laurate, isopropyl myristate, myristyl myristate, isocetyl myristate, octyldodecyl myristate, isopropyl palmitate, octyl palmitate, cetyl palmitate, isocetyl palmitate, isostearyl palmitate, butyl stearate, hexyldecyl stearate, isopropyl isostearate, isocetyl isostearate, decyl oleate, oleil oleate, octyldodecyl oleate, ethyl linoate, isopropyl linoate, cetyl lactate, myristyl lactate, cholesteryl hydroxystearate, dioctyldodecyl lauroylglutamate, and isopropyl lauroylsarcocinate.

Illustrative example of the natural plant and animal oil and the semi-synthetic oil include an avocado oil, an almond oil, an olive oil, a wheat germ oil, a sesame oil, a rice germ oil, a rice bran oil, a sugarcane wax, a sasanqua oil, a safflower oil, a cinnamon oil, squalane, squalene, a turtle oil, a soybean oil, a tea seed oil, a camellia oil, an evening primrose oil, a corn oil, a rapeseed oil, a Japanese tung oil, a germ oil, a sunflower oil, a grape seed oil, a jojoba oil, a macademia nut oil, a cotton seed oil, a coconut oil, a cured coconut oil, a tri-coconut fatty acid glyceride, a peanut oil, and an egg-yolk oil.

### [Paste composition]

The paste composition of the present invention contains an organopolysioloxane polymer (I) and an oil material (II). Specifically, an organopolysiloxane polymer (I), obtained after removal of an organic solvent if the addition polymerization is carried out by using the organic solvent, or after removal of a volatile component depending on the purpose even if an organic solvent is not used therein, is kneaded with an oil material (II) that is a liquid at 25°C, or with an additional oil material (II) if the addition polymerization is carried out in co-existence of a non-reactive silicone oil or an organic oil, thereby producing a paste composition swelled with the oil material.

The kneading method for obtaining the paste composition of the present invention, it is preferable that the kneading be carried out under a shear force after the organopolysiloxane polymer (I) and the oil material (II) that is a liquid at 25°C, the both being according to the present invention, are mixed, so that the paste composition having a smooth appearance may be obtained. Kneading under a shear force may be carried out by using, for example, a three-roll mill, a two-roll mill, a sand grinder, a colloid mill, a homogenizer, an ultramixer, a homomixer, and a homodisper, while a three-roll mill and a homodisper are preferable.

The mixing ratio of the oil material (II) to the organopolysiloxane polymer (I) of the present invention is preferably 1/20 to 20/1 (mass ratio), or more preferably 1/10 to 1/1. The paste composition with the mixing ratio of the oil material (II) to the organopolysiloxane polymer (I) being 1/20 to 20/1 (mass ratio) is preferable because the paste composition giving a silky and light feeling without poor spreading properties and without such feelings as an oily, a sticky, and an oil-film feeling can be obtained.

### [Cosmetic]

In addition, the present invention provides a cosmetic characterized in that the paste composition described above is contained therein. If a cosmetic contains the paste composition as described above, the cosmetic giving a silky and light feeling without poor spreading properties and without such feelings as an oily, a sticky, and an oil-film feeling can be obtained. The cosmetic of the present invention may contain, in addition to the paste composition mentioned above, at least one kind component, allowed as a cosmetic component, selected from the group consisting of (A) an oil material, (B) water, (C) a compound having an alcoholic hydroxy group, (D) a water-soluble or a water-swelling polymer, (E) a powder, (F) a surfactant, (G) a conventional silicone resin, and (H) a composition other than the paste composition of the present invention; the composition here comprising a crosslinking organopolysiloxane and a liquid oil that are in the public domain.

The oil material (A) may be in the form of any of a solid, a semi-solid, and a liquid. As to the liquid oil, the same liquid oil as those used in the paste composition of the present invention, other liquid oil, or a mixture of them may be used. Specific examples of the liquid oil are those as described before.

Illustrative example of the other oil component (A) includes a linseed oil, an insects wax, a perilla oil, a cacao butter, a kapok wax, a kaya oil, a carnauba wax, a lever oil, a candellila wax, a purified candellila wax, a beef tallow, a neats-foot oil, a beef bone fat, a cured beef tallow, an apricot kernel oil, a whale wax, a hydrogenated oil, a shea butter, a Chinese tung oil, a jojoba wax, a shellac wax, a pig fat, a horse fat, a bran wax, a persic oil, a palm oil, a palm kernel oil, a castor oil, a cured castor oil, a methyl ester of castor oil fatty acid, a bayberry wax, a bees wax, a mink oil, a meadowfoam seed oil, a mutton tallow, a cotton wax, a Japan wax, a Japan wax kernel oil, a montan wax, lanolin, liquid lanolin, reduced lanolin, lanolin alcohol, hard lanolin, lanolin acetate, lanolin alcohol acetate, isopropyl lanolin fatty acid, polyoxyethylene lanolin alcohol ether, polyoxyethylene lanolin alcohol acetate, polyethylene glycol lanolin fatty acid, and polyoxyethylene hydrogenated lanolin alcohol ether. Illustrative example of the hydrocarbon oil contained in the oil component (A) includes an ozocerite, a ceresin, a paraffin, a paraffin wax, a polyethylene wax, a polyethylene/polypropylene wax, a pristane, polyisobutylene, a microcrystalline wax, and vaselin. Illustrative example of the higher fatty acid contained in the oil component (A) includes lauric acid, myristic acid, palmitic acid, stearic acid, behenic acid, isostearic acid, and 12-hydroxystearic acid. Illustrative example of the higher alcohol contained in the oil component (A) includes an alcohol having preferably 6 or more, or more preferably 10 to 30 carbon atoms. Specific example of the foregoing higher alcohol includes lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyl tetradecynol, cholesterol, phytosterol, polyoxyethylene cholesterol ether, monostearyl glycerine ether (batyl alcohol), and monooleyl glyceryl ether (selachyl alcohol). Illustrative example of the fluorinated oil material contained in the oil component (A) includes perfluoro polyether, perfluoro decalin, and perfluoro octane.

Amount of the oil component (A) to be blended dependent on the form of the cosmetic of the present invention; but it preferably is chosen, as appropriate, in the range of 1 to 98% by mass relative to the totality of the cosmetic.

Amount of the water component (B) to be blended dependent on the form of the cosmetic of the present invention; but it preferably is chosen, as appropriate, in the range of 1 to 95% by mass relative to the totality of the cosmetic.

Illustrative example of the compound having an alcoholic hydroxy group (C) includes a lower alcohol preferably having 2 to 5 carbon atoms (a lower monovalent alcohol) such as ethanol and isopropanol and a sugar alcohol such as sorbitol and maltose. In addition, included are a sterol such as cholesterol, sitosterol, phytosterol, and lanosterol; and a polyvalent alcohol such as butylene glycol, propylene glycol, dibutylene glycol, and pentylene glycol. Amount of the component (C) to be blended is chosen preferably and appropriately in the range of 0.1 to 98% by mass relative to the totality of the cosmetic.

Illustrative example of the water-soluble or the water-swelling polymer (D) includes a polymer derived from plant such as an Arabia gum, tragacanth, galactan, a carob gum, a guar gum, a karaya gum, carrageenan, pectin, agar, quince seed (marmelo), starch (rice, corn, potato, wheat, and so on), algae colloid, a trant gum, and a locust bean gum; a polymer derived from microbial such as a xanthan gum, dextran, succinoglucan, and pullulan; a polymer derived from animal such as collagen, casein, albumin, and gelatin; a starch polymer such as carboxymethyl starch and methylhydroxypropyl starch; a cellulose polymer such as methyl cellulose, ethyl cellulose, methyl hydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose, and cellulose; an alginic acid polymer such as sodium alginate and propylene glycol alginate ester; a vinyl polymer such as polyvinyl methyl ether and carboxy vinyl polymer; a polyoxyethylene polymer; a polyoxyethylene polyoxypropylene copolymer; an acryl polymer such as sodium polyacrylate, polyethyl acrylate, polyacrylamide, and acryloyldimethyl taurate salt copolymer; other synthetic water-soluble polymer such as polyethyleneimine and an cationic polymer; and an inorganic water-soluble polymer such as bentonite, aluminum magnesium silicate, montomorillonite, beidellite, nontronite, saponite, hectorite, and anhydrous silicic acid. In addition, included are a film forming material such as polyvinyl alcohol and polyvinyl pyrrolidone. Amount of the component (D) to be blended is preferably in the range of 0.1 to 25% by mass relative to the totality of the cosmetic of the present invention.

As to the powder (E), any powder may be used regardless of its form (spherical, needle-like, platelike, and so on), its particle diameter (fumed, microparticle, pigment-class, and so on), and its particle structure (porous, non-porous, and so on), provided that the powder can be used in a usual cosmetic. Illustrative example of the powder includes an inorganic powder, an organic powder, a surfactant metal salt powder, a colored pigment, a pearl pigment, a metal powder pigment, and a natural dye.

Illustrative example of the inorganic powder includes a microparticle of titanium oxide, titanium mica, zirconium oxide, zinc oxide, cerium oxide, magnesium oxide, barium sulfate, calcium sulfate, magnesium sulfate, calcium carbonate, magnesium carbonate, talc, cleaved talc, mica, kaolin, sericite, white mica, synthetic mica, golden mica, red mica, black mica, lithia mica, silicic acid, silicon dioxide, fumed silica, hydrated silicon dioxide, aluminum silicate, magnesium silicate, aluminum magnesium silicate, calcium silicate, barium silicate, strontium silicate, a metal tungstate salt, hydroxy apatite, vermiculite, higilite, bentonite, montomorillonite, hectorite, zeolite, ceramics, dibasic calcium phosphate, alumina, aluminum hydroxide, boron nitride, boron nitride, and glass.

Illustrative example of the organic powder includes a polyamide, polyacrylic acid, a polyacrylate ester, a polyester, polyethylene, polypropylene, polystyrene, styrene-acrylic acid copolymer, divinyl benzene-styrene copolymer, a polyurethane, a vinyl resin, an urea resin, a melamine resin, bezoguanamine, polymethyl benzoguanamine, tetrafluoroethylene, a polymethacrylate methyl (such as polymethacrylate methyl), cellulose, silk, nylon, a phenolic resin, an epoxy resin, polycarbonate, a silicone elastomer particle, polymethyl silsesquioxane particle, and a powder obtained by coating surface of the silicone elastomer particle described in Japanese Patent No. 2832143 with polymethyl silsesquioxane.

Illustrative example of the surfactant metal salt powder includes a powder of zinc stearate, aluminum stearate, calcium stearate, magnesium stearate, zinc myristate, magnesium myristate, zinc cetylphosphate, calcium cetylphosphate, and sodium cetylphosphate zinc.

Illustrative example of the colored pigment includes an iorganic red pigment such as iron oxide, iron hydroxyide, and iron titanate; an inorganic brown pigment such as γ-iron oxide; an inorganic yellow pigment such as a yellow iron oxide and a yellow earth; an inorganic black pigment such as a black iron oxide and a carbon black; an inorganic purple pigment such as a manganese violet and a cobalt violet; an inorganic green pigment such as chromium hydroxyide, chromium oxide, cobalt oxide, and cobalt titanate; an inorganic blue pigment such as Prussian blue and azurite; a laked tar dye; and a laked natural dye.

Specific example of the pearl pigment includes a mica covered with titanium oxide, oxychloro bismuth, oxychloro bismuth covered with titanium oxide, a talc covered with titanium oxide, a fish scale foil, and a colored mica covered with titanium oxide. Illustrative example of the metal powder pigment includes an aluminum powder, a copper powder, and a stainless powder.

Illustrative example of the tar dye includes Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207. Illustrative example of the natural dye includes carminic acid, laccaic acid, carthamin, brazilin, and crocin.

Among these powders (E), preferable in the present invention are a silicone elastomer particle, a polymethyl silsesquioxane particle, and a powder obtained by coating surface of the silicone elastomer particle described in Japanese Patent No. 2832143 with polymethyl silsesquioxane; and in addition, any one of a powder having a fluorine group and a coloring agent or both. In addition, usable as the powder (E) are a powder obtained by hybridization of these powders to a degree not adversely affecting the effects of the present invention; a powder treated with a general oil material, a silicone oil, a fluorine-containing compound, a surfactant, and the like; a reactive organohydrogen polysiloxane; an organopolysiloxane having a hydrolysable alkoxysilane group; and a powder treated with an acryl-silicone copolymer having a hydrolysable silyl group; or, as appropriate, a mixture of one, or two or more of them.

Amount of the powder (E) to be blended is preferably 0.1 to 99% by mass relative to the totality of the cosmetic. Especially in the case of a powdery solid cosmetic, the amount thereof is preferably 80 to 99% by mass relative to the totality of the cosmetic.

As to the surfactant (F), there are an anionic, a cationic, a nonionic, and an amphoteric surfactant; and in the present invention, there is no particular restriction, and thus any of them may be used provided that the surfactant is used in a usual cosmetic.

Illustrative example of the anionic surfactant includes an alkyl sulfate salt, a polyoxyethylene alkyl ester sulfate salt, a polyoxyethylene alkyl phenyl ether sulfate salt, a N-acyl taurate salt, an alkylbenzene sulfonate salt, a polyoxyethylene alkyl phenyl ether sulfonate salt, an α-olefin sulfonate salt, an alkylnaphthalene sulfonate salt, an alkyl diphenyl ether disulfonate salt, a dialkyl sulfosuccinate salt, a monoalkyl sulfosuccinate salt, a polyoxyethylene alkyl ether sulfosuccinate salt, an fatty acid salt, a polyoxyethylene alkyl ether acetate salt, a N-acyl amino acid salt, an alkenylsuccinate salt, an alkylphosphate salt, a polyoxyethylene alkyl ether phosphate salt, a polystyrene sulfonate salt, a condensation product of a naphthalene sulfonic acid with formalin, a condensation product of an aromatic sulfonic acid with formalin, a carboxylic acid polymer, and a copolymer of styrene with an oxyalkylene acid anhydride.

Illustrative example of the cationic surfactant includes an alkyl trimethyl ammonium salt, a dialkyl dimethyl ammonium salt, a polyoxyethylene alkyl dimethyl ammonium salt, a dipolyoxyethylene alkyl methyl ammonium salt, a tripolyoxyethylene alkyl ammonium salt, an alkyl benzyl dimethyl ammonium salt, an alkyl pyridium salt, a monoalkyl amine salt, a monoalkylamide amine salt, and a cationic cellulose.

Illustrative example of the nonionic surfactant includes a polyoxyethylene alkyl ether, a polyoxyethylene polyoxypropylene alkyl ether, a polyoxyethylene alkylphenyl ether, a polyethylene glycol fatty acid ester, a sorbitan fatty acid ester, a polyoxyethylene sorbitan fatty acid ester, a polyoxyethyelene sorbit fatty acid ester, a glycerin fatty acid ester, a polyoxyethylene glycerin fatty acid ester, a polyglycerin fatty acid ester, a propylene glycol fatty acid ester, a polyoxyethylene castor oil, a polyoxyethylene hard castor oil, a fatty acid ester of a polyoxyethylene hard castor oil, a polyoxyethylene alkyl amine, a polyoxyethylene fatty acid amide, a polyoxyalkylene-modified organopolysiloxane, an organopolysiloxane co-modified with a polyoxyalkylene and an alkyl, a polyglycerin-modified organopolysiloxane (Japanese Patent No. 2613124), an alkyl-modified silicone having a branched silicone (Japanese Patent No. 3850202), an organopolysiloxane co-modified with a branched silicone polyoxyalkylene and a long chain alkyl (Japanese Patent No. 3724988), and an organopolysiloxane modified with a silicone-branched polyglycerin (Japanese Patent No. 3976226).

Illustrative example of the amphoteric surfactant includes a betaine, an aminocarboxylic acid salt, an imidazoline derivative, and an amide amine type.

Among these surfactants (F), a linear or a branched organopolysiloxane having a polyglycerin chain or a polyoxyalkylene chain in the molecule, or an organopolysiloxane co-modified with an alkyl, with the amount of a hydrophilic polyoxyethylene group or a hydrophilic polyglycerin group being 10 to 70% by mass in the molecule, is preferable. In addition, amount thereof to be blended is preferably 0.1 to 20% by mass, or particularly preferably 0.2 to 10% by mass, relative to the totality of the cosmetic.

Illustrative example of the conventional silicone resin (G) includes those, which are in the form of a gum or in a solid state at a normal temperature; and it is preferable the one uniformly soluble in decamethyl cyclopentasiloxane. The silicone resin in the form of a gum is a linear polysiloxane shown by the following general formula (4), wherein R⁵ is selected from a methyl group; an alkyl group having 6 to 20 carbon atoms; an amino-containing alkyl group, an fluorine-substituted alkyl group, and an alkyl group having a quaternary ammonium salt, the number of carbon atoms contained in these alkyl groups being 3 to 15; and the "g" is a number preferably in the range of 1000 to 20000, the "h" is a number preferably in the range of 1 to 5000, and (g+h) is a number preferably in the range of 2000 to 20000.

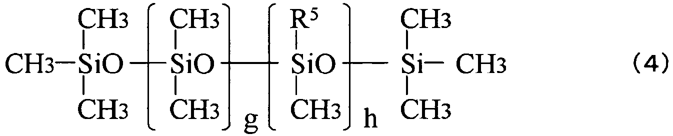

Illustrative example of the silicone resin in the solid state includes a silicone network compound with an arbitrary combination of a monoalkylsiloxy unit (M unit), a dialkylsiloxy unit (D unit), a trialkylsiloxy unit (T unit), and a four-functional siloxy unit (Q unit); namely a MQ resin, a MDQ resin, a MTQ resin, a MDTQ resin, a T resin, a TD resin, a TQ resin, a TDQ resin, and so on. Further included therein is an acryl silicone resin such as an acryl/silicone graft copolymer and an acryl/silicone block copolymer (specific example thereof includes KP-545: a cyclic organopolysiloxane solution of an acryl/silicone graft copolymer manufactured by Shin-Etsu Chemical Co., Ltd.). In addition, an acryl silicone resin having, within its molecular structure, at least one part selected from a pyrrolidone part, an alkyl long chain part, a polyoxyalkylene part, a fluoroalkyl part, an anionic part of a carboxylic acid and the like, may be used.

The conventional silicone resin (G) such as the silicone resin in the gum form, the silicone network compound, the acryl silicone resin, and so on may be used by dissolving in a low-viscosity silicone oil, a volatile silicone oil, or other solvent. Amount of the conventional silicone resin (G) to be used is, as the resin component, preferably 0.1 to 20% by mass, or more preferably 1 to 10% by mass, relative to the totality of the cosmetic.

The composition (H) comprising a crosslinking organopolysiloxane and a liquid oil, the both being in the public domain, is other than the paste composition of the present invention and illustrative example thereof includes those described in Japanese Examined Patent Publication No. H06-55897, Japanese Patent No. 2631772, Japanese Patent No. 3969728, Japanese Patent No. 4001342, and Japanese Patent No. 4490817. Illustrative example of the crosslinking organopolysiloxane swelled with a silicone oil contained in the component (H) includes KSG-15, 16, 17, 18, 21, 24, 210, and 710 (manufactured by Shin-Etsu Chemical Co., Ltd.); illustrative example of the crosslinking organopolysiloxane swelled with a hydrocarbon oil contained in the component (H) includes KSG-31, 32, 34, 310, 320, 340, 41, 42, 44, 810, 820, and 840 (manufactured by Shin-Etsu Chemical Co., Ltd.); and illustrative example of the crosslinking organopolysiloxane swelled with an ester oil contained in the component (H) includes KSG-33, 330, 43, and 830 (manufactured by Shin-Etsu Chemical Co., Ltd.). The composition (H) comprising a crosslinking organopolysiloxane and a liquid oil, the both being in the public domain, may be used with the amount in the range not to adversely affect the effects of the present invention; and thus, the amount thereof is preferably 0.1 to 50% by mass, or more preferably 1 to 30% by mass, relative to the totality of the cosmetic.

### [Other components]

The cosmetic of the present invention may be added with a component generally used in a usual cosmetic in the amount not to adversely affect the effects of the present invention; illustrative example thereof includes an oil-soluble gelation agent, an antiperspirant, a UV-absorber, a UV absorbing-scattering agent, a moisturizer, an antibacterial preservative, a fragrance, a salt, an antioxidant, a pH controller, a chelating agent, an algefacient, an anti-inflammatory agent, a skin care component (a skin-lightening agent, a cell activator, a rough-skin improver, a blood circulation promoter, a skin astringent agent, an antiseborrheic agent, and so on), a vitamin, an amino acid, a nucleic acid, a hormone, a clathrate compound, and a hair-immobilizing agent.

Illustrative example of the oil-soluble gelation agent includes a metal soap such as aluminum stearate, magnesium stearate, and zinc myristate; an amino acid derivative such as N-lauroyl-L-glutamic acid and α,γ-din-butyl amine; a dextrin fatty acid ester such as dextrin palmitate ester, dextrin stearate ester, and dextrin 2-ethylhexanoate palmitate ester; a sucrose fatty acid ester such as sucrose palmitate ester and sucrose stearate ester; a fructo-oligosaccharide fatty acid ester such as fructo-oligosaccharide stearate ester and fructo-oligosaccharide 2-ethylhexanoate ester; a benzylidene derivative of sorbitol such as monobenzylidene sorbitol and dibenzylidene sorbitol; an organic-modified clay mineral such as dimethyl benzyl dodecyl ammonium montomorillonite clay and dimethyl dioctadecyl ammonium montomorillonite clay.

Illustrative example of the antiperspirant includes aluminum chlorohydrate, aluminum chloride, aluminum sesquichlorohydrate, zirconyl hydroxy chloride, aluminum zirconium hydroxy chloride, and aluminum zirconium glycine complex.

Illustrative example of the UV-absorber includes a benzoic acid UV-absorber such as para-amino benzoic acid; an anthranilic acid UV-absorber such as methyl anthranilate; a salicylic acid UV-absorber such as methyl salicylate, octyl salicylate, and trimethylcyclohexyl salicylate; a cinnamic acid UV-absorber such as octyl para-methoxy cinnamate; a benzophenone UV-absorber such as 2,4-dihydroxybenzophenone; a urocanic acid UV-absorber such as ethyl urocanate; a dibenzoylmethane UV-absorber such as 4-t-butyl-4'-methoxy-dibenzoylmethane; phenyl benzimidazole sulfonic acid; and a triazine derivative. Illustrative example of the UV absorbing-scattering agent includes a particle, which absorbs and scatters a UV-beam, such as a titanium oxide microparticle, a titanium oxide microparticle containing an iron, a zinc oxide microparticle, a cerium oxide microparticle, and a composite material of them. A dispersed material obtained by dispersing the particle, which absorbs and scatters a UV-beam into an oil material prior to use may also be used.

Illustrative example of the moisturizer includes glycerin, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate salt, polyoxyethylene methyl glucoside, polyoxypropylene methyl glucoside, egg yolk lecithin, soybean lecithin, phosphatidyl choline, phosphatidyl ethanolamine, phosphatidyl serine, phosphatidyl glycerol, phosphatidyl inositol, and sphingo phospholipid.

Illustrative example of the antibacterial preservative includes a para-oxybenzoate alkyl ester, benzoic acid, sodium benzoate, sorbic acid, potassium sorbate, and phenoxy ethanol. Illustrative example of the antibacterial agent includes benzoic acid, salicylic acid, carbolic acid, sorbic acid, a para-oxybenzoic acid alkyl ester, p-chloro-m-cresol, hexachlorophene, benzalkonium chloride, chlorhexidine chloride, trichlorocarbanilide, a photosensitive element, phenoxy ethanol, and methyl isothiazoline.

Illustrative example of the fragrance includes a natural fragrance and a synthetic fragrance. Illustrative example of the natural fragrance includes a plant fragrance separated from a flower, a leaf, a trunk, and a fruit skin; and an animal fragrance such as musk and civet. Illustrative example of the synthetic fragrance includes a hydrocarbon such as monoterpene; an alcohol such as an aliphatic alcohol and an aromatic alcohol; an aldehyde such as a terpene aldehyde and an aromatic aldehyde; a ketone such as an alicyclic ketone; an ester such as a terpene ester; a lactone; a phenol; an oxide; a nitrogen-containing compound; and an acetal.

As to the salt, an inorganic salt, an organic salt, an amine salt, and an amino acid salt may be mentioned. Illustrative example of the inorganic salt includes a sodium, a potassium, a magnesium, a calcium an aluminum, a zirconium, and a zinc salt of an inorganic acid such as hydrochloric acid, sulfuric acid, carbonic acid, and nitric acid. Illustrative example of the organic salt includes a salt of an organic acid such as acetic acid, dehydroacetic acid, citric acid, malic acid, succinic acid, ascorbic acid, and stearic acid. Illustrative example of the amine salt includes a salt of triethanol amine. Illustrative example of the amino acid salt includes an amino acid salt such as a glutamate salt. In addition, a salt of hyaluronic acid and chondroitin sulfate, an aluminum zirconium glycine complex, and a neutralized salt obtained by neutralization of an acid and a base used in a cosmetic prescription may be used.

Illustrative example of the antioxidant includes tocopherol, p-t-butylphenol, butyl hydroxy anisole, dibutyl hydroxy toluene, and phytic acid.

Illustrative example of the pH-controller includes lactic acid, citric acid, glycolic acid, succinic acid, tartaric acid, DL-malic acid, potassium carbonate, sodium bicarbonate, and ammonium bicarbonate. Illustrative example of the chelating agent includes alanine, sodium edetate, sodium polyphosphate, sodium metaphosphate, and phosphoric acid. Illustrative example of the algefacient includes L-menthol and camphor. Illustrative example of the anti-inflammatory agent includes allantoin, glycyrrhizic acid and its salt, glycyrrhetinoic acid and stearyl glycyrrhetinate, tranexamic acid, and azulene.

Illustrative example of the skin care component includes a skin-lightening agent such as a placenta extract, arbutin, glutathione, and a saxifrage extract; a cell activator such as a royal jelly, a photosensitive element, a cholesterol derivative, and an extract from hemolysed blood of young calves; a rough-skin improver; a blood circulation promoter such as nonylic acid warenylamide, benzyl nicotinate ester, β-butoxyethyl nicotinate ester, capsaicin, zingerone, cantharides tincture, ichthammol, caffeine, tannic acid, α-borneol, nicotinic acid tocopherol, inositol hexanicotinate, cyclandelate, cinnarizine, tolazoline, acetyl choline, verapamil, cepharanthin, and γ-orizanol; a skin astringent agent such as zinc oxide and tannic acid; and an antiseborrheic agent such as sulfur and thianthrol.

Illustrative example of the vitamin includes a vitamin A such as a vitamin A oil, retinol, retinol acetate, and retinol palmitate; a vitamin B including a vitamin B₂ such as riboflavin, riboflavin butyrate, and a flavin adenine nucleotide, a vitamin B₆ such as pyridoxine hydrochloride salt, pyridoxine dioctanoate, and pyridoxine tripalmitate, a vitamin B₁₂ and its derivative, and vitamin B₁₅ and its derivative; a vitamin C such as L-ascorbic acid, L-ascorbic acid dipalmitate ester, sodium L-ascorbic-2-sulfate, and dicalcium L-ascorbic acid phosphate diester; a vitamin D such as ergocalciferol and cholecalciferol; a vitamin E such as α-tocopherol, β-tocopherol, γ-tocopherol, DL-α-tocopherol acetate, DL-α-tocopherol nicotinate, and DL-α-tocopherol succinate; a nicotinic acid such as nicotinic acid, benzyl nicotinate, and a nicotinic acid amide; vitamin H; vitamin P; a pantothenic acid such as calcium pantothenate, D-pantothenyl alcohol, pantothenyl ethyl ether, and acetyl pantothenyl ethyl ether; and biotin.

Illustrative example of the amino acid includes glycine, valine, leucine, isoleucine, serine, threonine, phenylalanine, arginine, lysine, aspartic acid, glutamic acid, cystine, cysteine, methionine, and tryptophan. Illustrative example of the nucleic acid includes deoxyribonucleic acid. Illustrative example of the hormone includes estradiol and ethinyl estradiol. Illustrative example of the clathrate compound includes cyclodextrin.

As to the hair-immobilizing agent, an amphoteric polymer, an anionic polymer, a cationic polymer, and a nonionic polymer may be mentioned; and illustrative example of the hair-immobilizing agent includes a polyvinyl pyrrolidone polymer such as polyvinyl pyrrolidone and vinyl pyrrolidone/vinyl acetate copolymer; an acidic vinyl ether polymer such as methyl vinyl ether/maleic anhydride alkyl half-ester copolymer; an acidic polyvinyl acetate polymer such as vinyl acetate/crotonic acid copolymer; an acidic acryl polymer such as a (meth)acrylic acid/alkyl (meth)acrylate copolymer and a (meth)acrylic acid/alkyl (meth)acrylate/alkyl acrylamide copolymer; and an amphoteric acryl polymer such as a N-methacryloylethyl-N,N-dimethyl ammonium/α-N-methylcarboxybetaine/alkyl (meth)acrylate copolymer and hydroxypropyl (meth)acrylate/butylaminoethyl methacrylate/acrylic acid octyl amide copolymer. In addition, a polymer derived from a nature such as cellulose or its derivative, keratin or its derivative, and collagen or its derivative may be used suitably.

The cosmetics of the present invention are those blended with the foregoing cosmetic components, and include a skin care cosmetic such as a milky lotion, a cream, a cleansing, a pack, an oil liquid, a massage material, a liquid cosmetic, a cleaning cosmetic, a deodorant, a hand cream, and a lip cream; a make-up cosmetic such as a make-up foundation, a white powder, a liquid foundation, an oil foundation, a rouge, an eye shadow, a mascara, an eye liner, an eye blow, and a lipstick; a hair cosmetic such as a shampoo, a rinse, a conditioner, a treatment, and a setting material; an antiperspirant; and a UV-protective cosmetic such as a sunscreen lotion and a sun-cut cream. These cosmetics may be in any form of a liquid, a lotion, a cream, a solid, a paste, a gel, a powder, pressed, multi-layered, a mousse, a spray, a stick, and so on.

### EXAMPLES

The present invention will be explained in more detail by showing Synthesis Examples of the paste composition of the present invention and Comparative Synthesis Examples thereof, and by Examples of the cosmetic including the paste composition of the present invention and Comparative Examples thereof; but the present invention is not limited to them. Meanwhile, viscosity was measured at 25°C by using a digital viscometer DV-II+Pro (manufactured by Brookfield Engineering Laboratories, Inc.).

### [Synthesis Example 1]

Into a reactor were charged 80.0 parts by mass of the methylhydrogen polysiloxane shown by the following formula (5), 20.0 parts by mass of the methylhydrogen polysiloxane shown by the following formula (6), 155.0 parts by mass of the methylvinyl polysiloxane shown by the following formula (7), and 85.0 parts by mass of dimethyl polysiloxane (viscosity of 6 mm²/second) as a non-reactive silicone oil; and then, after 0.09 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer (I).

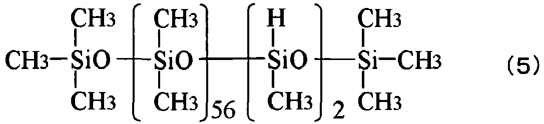

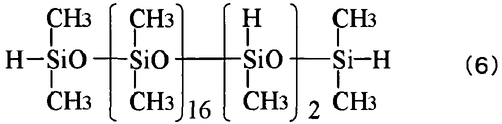

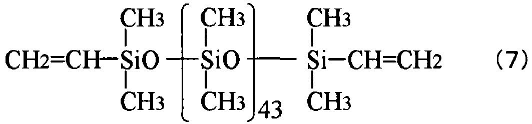

Then, 100 parts by mass of the product thus obtained and 275 parts by mass of dimethyl polysiloxane (viscosity of 6 mm²/second) as the oil material (II) were mixed and kneaded sufficiently by using a three-roll mill to obtain a paste composition having the viscosity of 423,000 mPa·s with the mass ratio of the organopolysiloxane polymer (I) to dimethyl polysiloxane being 20/80.

### [Synthesis Example 2]

Into a reactor were charged 80.0 parts by mass of the methylhydrogen polysiloxane shown by the formula (5), 20.0 parts by mass of the methylhydrogen polysiloxane shown by the formula (6), 137.0 parts by mass of the methylvinyl polysiloxane shown by the formula (7), 59.0 parts by mass of the methylvinyl polysiloxane shown by the following formula (8), and 127.0 parts by mass of dimethyl polysiloxane (viscosity of 6 mm²/second) as a non-reactive silicone oil; and then, after 0.10 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer (I).

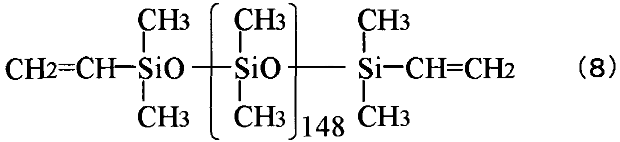

Then, 100 parts by mass of the product thus obtained and 289 parts by mass of dimethyl polysiloxane (viscosity of 6 mm²/second) as the oil material (II) were mixed and kneaded sufficiently by using a three-roll mill to obtain a paste composition having the viscosity of 407,000 mPa·s with the mass ratio of the organopolysiloxane polymer (I) to dimethyl polysiloxane being 18/82.

### [Synthesis Example 3]

Into a reactor were charged 80.0 parts by mass of the methylhydrogen polysiloxane shown by the formula (5), 20.0 parts by mass of the methylhydrogen polysiloxane shown by the formula (6),71.0 parts by mass of the methylvinyl polysiloxane shown by the formula (8), 31.0 parts by mass of the methylvinyl polysiloxane shown by the following formula (9), and 86.0 parts by mass of dimethyl polysiloxane (viscosity of 6 mm²/second) as a non-reactive silicone oil; and then, after 0.07 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer (I).

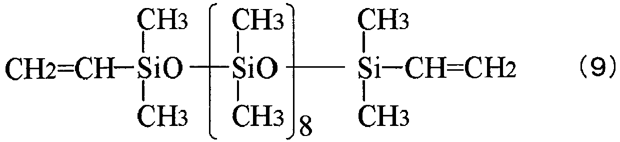

Then, 100 parts by mass of the product thus obtained and 289 parts by mass of dimethyl polysiloxane (viscosity of 6 mm²/second) as the oil material (II) were mixed and kneaded sufficiently by using a three-roll mill to obtain a paste composition having the viscosity of 416,000 mPa·s with the mass ratio of the organopolysiloxane polymer (I) to dimethyl polysiloxane being 18/82.

### [Synthesis Example 4; Reference example]

Into a reactor were charged 60.0 parts by mass of the methylhydrogen polysiloxane shown by the following formula (10), 40.0 parts by mass of the methylhydrogen polysiloxane shown by the following formula (11), 236.0 parts by mass of the methylvinyl polysiloxane shown by the following formula (12), and 84.0 parts by mass of dimethyl polysiloxane (viscosity of 6 mm²/second) as a non-reactive silicone oil; and then, after 0.10 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer (I).

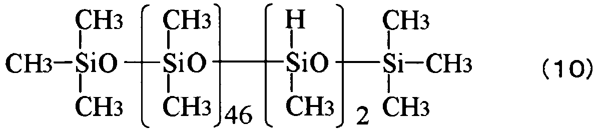

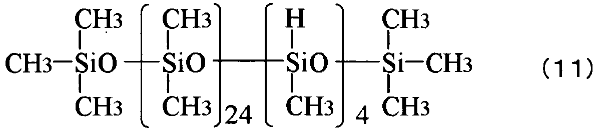

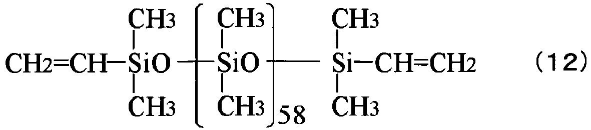

Then, 100 parts by mass of the product thus obtained and 300 parts by mass of dimethyl polysiloxane (viscosity of 6 mm²/second) as the oil material (II) were mixed and kneaded sufficiently by using a three-roll mill to obtain a paste composition having the viscosity of 419,000 mPa·s with the mass ratio of the organopolysiloxane polymer (I) to dimethyl polysiloxane being 20/80.

### [Synthesis Example 5]

Into a reactor were charged 70.0 parts by mass of the methylhydrogen polysiloxane shown by the formula (10), 30.0 parts by mass of the methylhydrogen polysiloxane shown by the following formula (13), and 130.0 parts by mass of the methylvinyl polysiloxane shown by the formula (12); and then, after 0.06 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer (I).

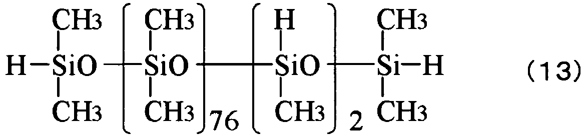

Then, 100 parts by mass of the product thus obtained and 300 parts by mass of dimethyl polysiloxane (viscosity of 6 mm²/second) as the oil material (II) were mixed and kneaded sufficiently by using a three-roll mill to obtain a paste composition having the viscosity of 435,000 mPa·s with the mass ratio of the organopolysiloxane polymer (I) to dimethyl polysiloxane being 25/75.

### [Synthesis Example 6]

Into a reactor were charged 70.0 parts by mass of the methylhydrogen polysiloxane shown by the formula (10), 30.0 parts by mass of the methylhydrogen polysiloxane shown by the formula (13), 130.0 parts by mass of the methylvinyl polysiloxane shown by the formula (12), and 100.0 parts by mass of dimethyl polysiloxane (viscosity of 1.5 mm²/second) as a non-reactive silicone oil; and then, after 0.08 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer (I).

Then, 100 parts by mass of the product thus obtained and 250 parts by mass of dimethyl polysiloxane (viscosity of 1.5 mm²/second) as the oil material (II) were mixed and kneaded sufficiently by using a three-roll mill to obtain a paste composition having the viscosity of 427,000 mPa·s with the mass ratio of the organopolysiloxane polymer (I) to dimethyl polysiloxane being 20/80.

### [Synthesis Example 7]

Into a reactor were charged 80.0 parts by mass of the methylhydrogen polysiloxane shown by the formula (5), 20.0 parts by mass of the methylhydrogen polysiloxane shown by the formula (6), 71.0 parts by mass of the methylvinyl polysiloxane shown by the formula (8), 31.0 parts by mass of the methylvinyl polysiloxane shown by the formula (9), and 165.0 parts by mass of dimethyl polysiloxane (viscosity of 1.5 mm²/second) as a non-reactive silicone oil; and then, after 0.09 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer (I).

Then, 100 parts by mass of the product thus obtained and 265 parts by mass of dimethyl polysiloxane (viscosity of 1.5 mm²/second) as the oil material (II) were mixed and kneaded sufficiently by using a three-roll mill to obtain a paste composition having the viscosity of 397,000 mPa·s with the mass ratio of the organopolysiloxane polymer (I) to dimethyl polysiloxane being 15/85.

### [Synthesis Example 8]

Into a reactor were charged 80.0 parts by mass of the methylhydrogen polysiloxane shown by the formula (10), 20.0 parts by mass of the methylhydrogen polysiloxane shown by the following formula (14), and 117.0 parts by mass of the methylvinyl polysiloxane shown by the formula (12); and then, after 0.05 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer (I).

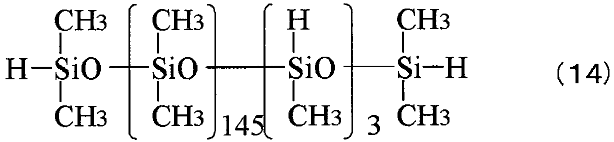

Then, 100 parts by mass of the product thus obtained and 565 parts by mass of decamethyl cyclopentasiloxane (viscosity of 4 mm²/second) as the oil material (II) were mixed and kneaded sufficiently by using a three-roll mill to obtain a paste composition having the viscosity of 403,000 mPa·s with the mass ratio of the organopolysiloxane polymer (I) to decamethyl cyclopentasiloxane being 15/85.

### [Synthesis Example 9; Reference example]

Into a reactor were charged 10.0 parts by mass of the methylhydrogen polysiloxane shown by the formula (13), 90.0 parts by mass of the methylhydrogen polysiloxane shown by the following formula (15), 84.8 parts by mass of the methylvinyl polysiloxane shown by the formula (7), 21.2 parts by mass of the methylvinyl polysiloxane shown by the formula (9), and 88.3 parts by mass of dimethyl polysiloxane (viscosity of 6 mm²/second); and then, after 0.09 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the crosslinking organopolysiloxane polymer.

Then, 100 parts by mass of the product thus obtained and 180 parts by mass of dimethyl polysiloxane (viscosity of 6 mm²/second) were mixed and kneaded sufficiently by using a three-roll mill to obtain a paste composition having the viscosity of 480,000 mPa·s with the mass ratio of the crosslinking organopolysiloxane polymer to dimethyl polysiloxane being 25/75.

### [Comparative Synthesis Example 1]

Into a reactor were charged 80.0 parts by mass of the methylhydrogen polysiloxane shown by the formula (5), 20.0 parts by mass of the methylhydrogen polysiloxane shown by the formula (6), 36.0 parts by mass of the methylvinyl polysiloxane shown by the formula (9), and 45.0 parts by mass of dimethyl polysiloxane (viscosity of 6 mm²/second) as a non-reactive silicone oil; and then, after 0.05 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer.

Then, 100 parts by mass of the product thus obtained and 200 parts by mass of dimethyl polysiloxane (viscosity of 6 mm²/second) as the oil material were mixed and kneaded sufficiently by using a three-roll mill to obtain a paste composition having the viscosity of 412,000 mPa·s with the mass ratio of the organopolysiloxane polymer to dimethyl polysiloxane being 25/75.

### [Comparative Synthesis Example 2]

Into a reactor were charged 60.0 parts by mass of the methylhydrogen polysiloxane shown by the formula (10), 40.0 parts by mass of the methylhydrogen polysiloxane shown by the formula (11), 41.0 parts by mass of the methylvinyl polysiloxane shown by the formula (9), and 35.0 parts by mass of dimethyl polysiloxane (viscosity of 6 mm²/second) as a non-reactive silicone oil; and then, after 0.04 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer.

Then, 100 parts by mass of the product thus obtained and 185 parts by mass of dimethyl polysiloxane (viscosity of 6 mm²/second) as the oil material were mixed and kneaded sufficiently by using a three-roll mill to obtain a paste composition having the viscosity of 414,000 mPa·s with the mass ratio of the organopolysiloxane polymer to dimethyl polysiloxane being 28/72.

### [Comparative Synthesis Example 3]

Into a reactor were charged 70.0 parts by mass of the methylhydrogen polysiloxane shown by the following formula (15), 30.0 parts by mass of the methylhydrogen polysiloxane shown by the formula (11), 51.0 parts by mass of the methylvinyl polysiloxane shown by the formula (9), and 100.0 parts by mass of dimethyl polysiloxane (viscosity of 6 mm²/second) as a non-reactive silicone oil; and then, after 0.06 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer.

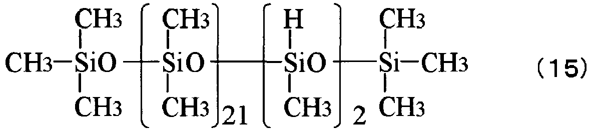

Then, 100 parts by mass of the product thus obtained and 140 parts by mass of dimethyl polysiloxane (viscosity of 6 mm²/second) as the oil material were mixed and kneaded sufficiently by using a three-roll mill to obtain a paste composition having the viscosity of 424,000 mPa·s with the mass ratio of the organopolysiloxane polymer to dimethyl polysiloxane being 25/75.

### [Comparative Synthesis Example 4]

Into a reactor were charged 70.0 parts by mass of the methylhydrogen polysiloxane shown by the formula (15), 30.0 parts by mass of the methylhydrogen polysiloxane shown by the formula (11), 222.0 parts by mass of the methylvinyl polysiloxane shown by the formula (7), and 81.0 parts by mass of dimethyl polysiloxane (viscosity of 6 mm²/second) as a non-reactive silicone oil; and then, after 0.10 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer.

Then, 100 parts by mass of the product thus obtained and 185 parts by mass of dimethyl polysiloxane (viscosity of 6 mm²/second) as the oil material were mixed and kneaded sufficiently by using a three-roll mill to obtain a paste composition having the viscosity of 409,000 mPa·s with the mass ratio of the organopolysiloxane polymer to dimethyl polysiloxane being 28/72.

### [Comparative Synthesis Example 5]

Into a reactor were charged 20.0 parts by mass of the methylhydrogen polysiloxane shown by the formula (5), 80.0 parts by mass of the methylhydrogen polysiloxane shown by the formula (15), 28.0 parts by mass of the methylvinyl polysiloxane shown by the formula (9), 42.0 parts by mass of the methylvinyl polysiloxane shown by the formula (7), and 43.0 parts by mass of dimethyl polysiloxane (viscosity of 6 mm²/second) as a non-reactive silicone oil; and then, after 0.05 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer.

Then, 100 parts by mass of the product thus obtained and 220 parts by mass of dimethyl polysiloxane (viscosity of 6 mm²/second) as the oil material were mixed and kneaded sufficiently by using a three-roll mill to obtain a paste composition having the viscosity of 422,000 mPa·s with the mass ratio of the organopolysiloxane polymer to dimethyl polysiloxane being 25/75.

### [Comparative Synthesis Example 6]

Into a reactor were charged 100.0 parts by mass of the methylhydrogen polysiloxane shown by the formula (15), 42.0 parts by mass of the methylvinyl polysiloxane shown by the formula (9), and 61.0 parts by mass of dimethyl polysiloxane (viscosity of 6 mm²/second) as a non-reactive silicone oil; and then, after 0.05 parts by mass of the divinyl tetramethyl disiloxane solution containing 2% by mass of chloroplatinic acid as an addition polymerization catalyst was added thereinto, the resulting mixture was stirred with maintaining the inner temperature thereof at 70 to 80°C for two hours to obtain a product containing the organopolysiloxane polymer.

Then, 100 parts by mass of the product thus obtained and 180 parts by mass of dimethyl polysiloxane (viscosity of 6 mm²/second) as the oil material were mixed and kneaded sufficiently by using a three-roll mill to obtain a paste composition having the viscosity of 428,000 mPa·s with the mass ratio of the organopolysiloxane polymer to dimethyl polysiloxane being 25/75.

Each compositional pattern of the respective paste compositions in Synthesis Examples 1 to 8 and Comparative Synthesis Examples 1 to 6 are shown in Table 1. Unit of the values in the formulae is shown by parts by mass. The hydrogen polysiloxanes or the vinyl polysiloxanes shown by the formulae (6), (9), (11), and (15) are those hydrogen polysiloxanes or vinyl polysiloxanes satisfying none of (a+b)≥30, (c+d)≥30, and (e+f)≥30. In Comparative Synthesis Example 5, the example is shown for the case that the hydrogen polysiloxane or the vinyl polysiloxane satisfying any of (a+b)≥30, (c+d)≥30, and (e+f)≥30 is contained only less than 40% by mass relative to the total mass of the polysiloxanes shown by the general formulae (1) to (3).

**[Table 1]**

| | Structure | | Synthesis Examples | | | | | | | | | Comparative Synthesis Examples | | | | | |
|---|---|---|---|---|---|---|---|---|---|---|---|---|---|---|---|---|---|
| | General formula | Degree of polymerization | 1 | 2 | 3 | 4* | 5 | 6 | 7 | 8 | 9* | 1 | 2 | 3 | 4 | 5 | 6 |
| Formula 5 | (2) | 58 | 80 | 80 | 80 | | | | 80 | | | 80 | | | | 20 | |
| Formula 6 | (1) | 18 | 20 | 20 | 20 | | | | 20 | | | 20 | | | | | |
| Formula 10 | (2) | 48 | | | | 60 | 70 | 70 | | 80 | | | 60 | | | | |
| Formula 11 | (2) | 28 | | | | 40 | | | | | | | 40 | 30 | 30 | | |
| Formula 13 | (1) | 78 | | | | | 30 | 30 | | | 10 | | | | | | |
| Formula 14 | (1) | 148 | | | | | | | | 20 | | | | | | | |
| Formula 15 | (2) | 23 | | | | | | | | | 90 | | | 70 | 70 | 80 | 100 |
| Formula 7 | (3) | 43 | 155 | 137 | | | | | | | 85 | | | | 222 | 42 | |
| Formula 8 | (3) | 148 | | 59 | 71 | | | | 71 | | | | | | | | |
| Formula 9 | (3) | 8 | | | 31 | | | | 31 | | 21 | 36 | 41 | 51 | | 28 | 42 |
| Formula 12 | (3) | 58 | | | | 236 | 130 | 130 | | 117 | | | | | | | |
| Number of the kind having 30 or more degree of polymerization | | | 2 | 3 | 2 | 2 | 3 | 3 | 2 | 3 | 2 | 1 | 1 | 0 | 1 | 2 | 0 |
| Ratio of the kinds having 30 or more degree of polymerization | | | 92% | 93% | 75% | 88% | 100% | 100% | 75% | 100% | 46% | 59% | 43% | 0% | 69% | 36% | 0% |

| | | | | | | | | | | | | | | | | | |
|---|---|---|---|---|---|---|---|---|---|---|---|---|---|---|---|---|---|
| * Reference examples | | | | | | | | | | | | | | | | | |

Evaluation of feeling, when each of the paste compositions of Synthesis Examples 1 to 8 and Comparative Synthesis Examples 1 to 6 was applied to skins of 10 expert panelists, was made according to the criteria shown in Table 2. Judgment was made according to the criteria shown in Table 3 based on the average value obtained. Judgment results thereof are shown in Table 4.

### [Evaluation Criteria]

**[Table 2]**

| Score | Items | | | |
|---|---|---|---|---|
| | Powdery feeling | Greasiness | Stickiness | Spreading |
| 5 | Strongly felt | None | None | Good |
| 4 | Felt | Very little | Very little | Comparatively good |
| 3 | Somewhat felt | Little | Little | Fair |
| 2 | Slightly felt | Some | Some | Comparatively bad |
| 1 | Not felt | Strong | Strong | Bad |

### [Judgment Criteria]

**[Table 3]**

| Criteria | Judgment |
|---|---|
| Obtained average score of 4.5 or more | Excellent |
| Obtained average score of 3.5 or more and less than 4.5 | Good |
| Obtained average score of 2.5 or more and less than 3.5 | Fair |
| Obtained average score of 1.5 or more and less than 2.5 | Poor |
| Obtained average score of less than 1.5 | Bad |

### [Judgment Results]

**[Table 4]**

| | Items | | | | |
|---|---|---|---|---|---|
| | | Powdery feeling | Greasiness | Stickiness | Spreading |
| Synthesis Examples | 1 | Excellent | Excellent | Excellent | Excellent |
| | 2 | Excellent | Excellent | Excellent | Excellent |
| | 3 | Excellent | Excellent | Excellent | Excellent |
| | 4* | Good | Excellent | Excellent | Excellent |
| | 5 | Excellent | Excellent | Excellent | Excellent |
| | 6 | Excellent | Excellent | Excellent | Excellent |
| | 7 | Excellent | Excellent | Excellent | Excellent |
| | 8 | Excellent | Excellent | Excellent | Excellent |
| | 9* | Good | Good | Excellent | Excellent |
| Comparative Synthesis Examples | 1 | Poor | Fair | Good | Excellent |
| | 2 | Poor | Poor | Good | Excellent |
| | 3 | Poor | Poor | Good | Excellent |
| | 4 | Poor | Poor | Good | Excellent |
| | 5 | Fair | Fair | Good | Excellent |
| | 6 | Bad | Bad | Good | Excellent |

| | | | | | |
|---|---|---|---|---|---|
| * Reference examples | | | | | |

As shown in Table 4, the paste compositions of Synthesis Examples 1 to 8 according to the present invention do not give a greasy feeling as compared with the paste compositions of Comparative Synthesis Examples 1 to 6, but give a powdery feeling even though a spherical powder is not used. In other words, according to the paste composition of the present invention, because the organopolysiloxane polymer (I) shows a high thickening effect to the oil material (II) such as an silicone oil thereby enhancing a blending stability of the oil material, it is shown that the paste composition containing them has a silky and light feeling without poor spreading properties and without such feelings as an oily, a sticky, and an oil-film feeling.

Then, Example of each cosmetic containing the paste composition of the present invention that contains the organopolysiloxane composition (I) and the oil material (II) will be shown.

### [Example 1: w/o milky lotion]

| (Components) | (parts by mass) |
|---|---|
| 1. Paste composition obtained in Synthesis Example 1 | 15.0 |
| 2. Dimethyl polysiloxane (viscosity: 6 mm²/second) | 12.0 |
| 3. Decamethyl cyclopentasiloxane | 10.0 |
| 4. Glyceryl trioctanoate | 5.0 |
| 5. Polyether-modified silicone (note 1) | 3.0 |
| 6. 1,3-butanediol | 5.0 |
| 7. Preservative | appropriate amount |
| 8. Fragrance | appropriate amount |
| 9. Purified water | 50.0 |

| | |
|---|---|
| (note 1: KF-6017, manufactured by Shin-Etsu Chemical Co., Ltd.) | |

### (Preparation method)

A: Components 1 to 5 were uniformly mixed by agitation.
B: Components 6 to 9 were uniformly mixed, and then added into A; and then, they were mixed by agitation to obtain an emulsion.

### (Result)

The emulsion thus obtained gave a w/o milky lotion with a shiny look, and with a wide and light spreading feeling and a refreshing feeling without stickiness.

### [Example 2: o/w cream]

| | |
|---|---|
| (Components) | (parts by mass) |
| 1. Paste composition obtained in Synthesis Example 2 | 8.0 |
| 2. Crosslinking methyl phenyl polysiloxane (note 2) | 2.0 |
| 3. Isotridecyl isononanoate | 5.0 |
| 4. Dipropylene glycol | 7.0 |
| 5. Glycerin | 5.0 |
| 6. 2% Methyl cellulose solution (note 3) | 7.0 |
| 7. Polyacrylamide emulsifier (note 4) | 2.0 |
| 8. Guanine | 1.0 |
| 9. Preservative | appropriate amount |
| 10. Fragrance | appropriate amount |
| 11. Purified water | 63.0 |

| | |
|---|---|
| (note 2: KSG-18, manufactured by Shin-Etsu Chemical Co., Ltd.) (note 3: Metolose SM-4000, manufactured by Shin-Etsu Chemical Co., Ltd.) (note 4: Sepigel 305, manufactured by SEPPIC S. A.) | |

### (Preparation method)

A: Components 3 to 11 were uniformly mixed by agitation.
B: Components 1 to 2 were uniformly mixed, and then added into A; and then, they were mixed by agitation to obtain an emulsion.

### (Result)

The emulsion thus obtained gave a fine o/w cream with a wide and light spreading feeling and a refreshing use feeling without stickiness and greasiness, and in addition, with good durability and stability.

### [Example 3: w/o cream]

| (Components) | (parts by mass) |
|---|---|
| 1. Paste composition obtained in Synthesis Example 8 | 5.0 |
| 2. Dimethyl polysiloxane (viscosity: 6 mm²/second) | 10.0 |
| 3. Crosslinking polyether-modified silicone (note 5) | 2.0 |
| 4. Polyether-modified branched silicone (note 6) | 0.5 |
| 5. Dipropylene glycol | 10.0 |
| 6. Sodium citrate | 0.2 |
| 7. Ethanol | 5.0 |
| 8. Preservative | appropriate amount |
| 9. Fragrance | appropriate amount |
| 10. Purified water | 67.3 |

| | |
|---|---|
| (note 5: KSG-210, manufactured by Shin-Etsu Chemical Co., Ltd.) (note 6: KF-6028, manufactured by Shin-Etsu Chemical Co., Ltd.) | |

### (Preparation method)

A: Components 1 to 4 were uniformly mixed by agitation.
B: Components 5 to 10 were uniformly mixed, and then added into A; and then, they were mixed by agitation to obtain an emulsion.

### (Result)

The emulsion thus obtained gave a w/o cream with a wide and light spreading feeling and a vivid, refreshing use feeling without greasiness and stickiness.

### [Example 4: powder foundation]

| (Components) | (parts by mass) |
|---|---|
| 1. Paste composition obtained in Synthesis Example 3 | 6.0 |
| 2. Acrylate/dimethyl silicone copolymer (note 7) | 3.0 |
| 3. Squalane | 3.0 |
| 4. Mica treated with acryl silicone (note 8) | 50.0 |
| 5. Talc treated with triethoxy caprylyl (note 9) | 24.0 |
| 6. Titanium oxide treated with triethoxy alkly silicone (note 10) | 9.0 |
| 7. Hybrid silicone composite powder (note 11) | 2.0 |
| 8. Spherical polymethyl silsesquioxane powder (note 12) | 3.0 |
| 9. Pigment treated with triethoxy alkyl silicone (note 13) | appropriate amount |
| 10. Preservative | appropriate amount |
| 11. Fragrance | appropriate amount |

| | |
|---|---|
| (note 7: KP-561P, manufactured by Shin-Etsu Chemical Co., Ltd.) (note 8: Mica treated with KP-574, manufactured by Shin-Etsu Chemical Co., Ltd.) (note 9: Talc treated with AES-3083, manufactured by Shin-Etsu Chemical Co., Ltd.) (note 10: Titanium oxide treated with KF-9909, manufactured by Shin-Etsu Chemical Co., Ltd.) (note 11: KSP-300, manufactured by Shin-Etsu Chemical Co., Ltd.) (note 12: KMP-590, manufactured by Shin-Etsu Chemical Co., Ltd.) (note 13: Pigment treated with KF-9909, manufactured by Shin-Etsu Chemical Co., Ltd.) | |

### (Preparation method)

A: Components 4 to 10 were uniformly mixed.
B: Components 1 to 3 were uniformly mixed, and then added into A; and then, they were mixed.
C: After component 11 was added thereinto, the resulting mixture was press molded in a mold to obtain a powder foundation.

### (Result)

The powder foundation thus obtained showed excellent adhesion with a light and wide spreading feeling without stickiness.

### [Example 5: w/o cream foundation]

| (Components) | (parts by mass) |
|---|---|
| 1. Paste composition obtained in Synthesis Example 6 | 8.0 |
| 2. Crosslinking polyether-modified silicone (note 14) | 3.0 |
| 3. Polyether-modified silicone (note 15) | 1.0 |
| 4. Dimethyl polysiloxane (viscosity: 6 mm²/second) | 3.0 |
| 5. Decamethyl pentasiloxane | 9.0 |
| 6. Glyceryl trioctanoate | 5.0 |
| 7. Neopentyl glycol dioctanoate | 2.0 |
| 8. Spherical polymethyl silsesquioxane powder (note 16) | 1.5 |
| 9. Branched silicone co-modified with polyglycerin and alkyl (note 17) | 2.0 |
| 10. Pigment treated with triethoxy alkyl silicone (note 18) | 5.0 |
| 11. Pentylene glycol | 5.0 |
| 12. Sodium chloride | 0.5 |
| 13. Preservative | appropriate amount |
| 14. Fragrance | appropriate amount |
| 15. Purified water | 55.0 |

| | |
|---|---|
| (note 14: KSG-210, manufactured by Shin-Etsu Chemical Co., Ltd.) (note 15: KF-6017, manufactured by Shin-Etsu Chemical Co., Ltd.) (note 16: KMP-590, manufactured by Shin-Etsu Chemical Co., Ltd.) (note 17: KF-6105, manufactured by Shin-Etsu Chemical Co., Ltd.) (note 18: Pigment treated with KF-9909, manufactured by Shin-Etsu Chemical Co., Ltd.) | |

### (Preparation method)

A: Components 1 to 4 and a part of component 5 were uniformly mixed with components 6 to 8 by agitation.
B: Components 9 to 10 and the rest of component 5 were uniformly mixed by agitation.
C: Components 11 to 13 and 15 were uniformly mixed.
D: C was added into A, and then they were mixed by agitation for emulsification.
E: Component 14 and B were added into D, and then they were mixed by agitation.

### (Result)

The cream foundation thus obtained gave a w/o cream foundation with a light and wide spreading feeling, good pigment dispersion, and excellent adhesion, and without greasiness and stickiness.

### [Example 6: eye shadow]

| (Components) | (parts by mass) |
|---|---|
| 1. Sericite | 45.0 |
| 2. Mica | 12.0 |
| 3. Talc | 12.0 |
| 4. Titanium oxide | 10.0 |
| 5. Titanium oxide microparticle | 5.0 |
| 6. Magnesium stearate | 3.0 |
| 7. Pigment | appropriate amount |
| 8. Octyl dodecanol | 3.0 |
| 9. Dimethyl polysiloxane (viscosity: 6 mm²/second) | 4.0 |
| 10. Paste composition obtained in Synthesis Example 7 | 6.0 |
| 11. Preservative | appropriate amount |
| 12. Fragrance | appropriate amount |

### (Preparation method)

A: Components 8 to 11 were uniformly mixed.
B: Components 1 to 7 were uniformly mixed, and then added into A; and then, they were mixed.
C: Component 12 was added into B.

### (Result)

The eye shadow thus obtained was excellent in spreading, adhesion, and cosmetic durability.

### [Example 7: cream eye color]

| (Components) | (parts by mass) |
|---|---|
| 1. Paste composition obtained in Synthesis Example 5 | 3.5 |
| 2. Paste composition obtained in Synthesis Example 7 | 5.0 |
| 3. Polyether-modified branched siloxane (note 19) | 2.0 |
| 4. Dimethyl polysiloxane (viscosity: 6 mm²/second) | 6.5 |
| 5. Decamethyl pentasiloxane | 26.6 |
| 6. Triethyl hexanoin | 5.0 |
| 7. Organic-modified bentonite | 1.2 |
| 8. Acrylate/dimethyl silicone copolymer (note 20) | 1.5 |
| 9. Pigment treated with triethoxy alkyl silicone (note 21) | 5.0 |
| 10. Dipropylene glycol | 5.0 |
| 11. Sodium citrate | 0.2 |
| 12. Preservative | appropriate amount |
| 13. Fragrance | appropriate amount |
| 14. Purified water | 38.5 |

| | |
|---|---|
| (note 19: KF-6028, manufactured by Shin-Etsu Chemical Co., Ltd.) (note 20: KP-575, manufactured by Shin-Etsu Chemical Co., Ltd.) (note 21: Pigment treated with KF-9909, manufactured by Shin-Etsu Chemical Co., Ltd.) | |

### (Preparation method)

A: Components 1 to 4 and a part of component 5 were uniformly mixed with components 6 to 7 by agitation.
B: Components 10 to 12 and component 14 were mixed by agitation.
C: Components 8 to 9 and the rest of component 5 were uniformly mixed.
D: C was added into A, and then they were mixed by agitation for emulsification.
E: Component 13 and B were added into D, and then they were mixed by agitation.

### (Result)

The cream eye color thus obtained was excellent in adhesion and pigment dispersion with a light and wide spreading feeling and without greasiness and stickiness.

### [Example 8: roll-on type antiperspirant]

| (Components) | (parts by mass) |
|---|---|
| 1. Paste composition obtained in Synthesis Example 8 | 20.0 |
| 2. Crosslinking polyether-modified silicone (note 22) | 20.0 |
| 3. Crosslinking dimethyl polysiloxane (note 23) | 15.0 |
| 4. Dimethyl polysiloxane (viscosity: 6 mm²/second) | 10.0 |
| 5. Decamethyl pentasiloxane | 15.0 |
| 6. Aluminum zirconium tetrachlorohydrate | 20.0 |
| 7. Fragrance | appropriate amount |

| | |
|---|---|
| (note 22: KSG-240, manufactured by Shin-Etsu Chemical Co., Ltd.) (note 23: KSG-15, manufactured by Shin-Etsu Chemical Co., Ltd.) | |

### (Preparation method)

A: Components 1 to 5 were uniformly mixed.
B: Components 6 to 7 were added into A; and then, they were mixed for dispersion.

### (Result)

The roll-on type antiperspirant thus obtained had a light and wide spreading property with a clean and cool feeling and without stickiness.

### [Example 9: sun-cut cream]

| (Components) | (parts by mass) |
|---|---|
| 1. Zinc oxide treated with triethoxy alkyl silicone (note 24) | 20.0 |
| 2. Silicone co-modified with alkyl and polyglycerin (note 25) | 12.0 |
| 3. Decamethyl cyclopentasiloxane | 20.0 |
| 4. Neopentyl glycol dioctanoate | 7.0 |
| 5. Crosslinking silicone co-modified with alkyl and polyether (note 26) | 2.0 |
| 6. Paste composition obtained in Synthesis Example | 5 5.0 |
| 7. Branched silicone co-modified with alkyl and polyether (note 27) | 1.0 |
| 8. Octyl methoxycinnamate | 3.0 |
| 9. Sodium citrate | 0.2 |
| 10. Dipropylene glycol | 3.0 |
| 11. Preservative | appropriate amount |
| 12. Fragrance | appropriate amount |
| 13. Purified water | 26.8 |

| | |
|---|---|
| (note 24: Zinc oxide treated with KF-9909, manufactured by Shin-Etsu Chemical Co., Ltd.) (note 25: KF-6105, manufactured by Shin-Etsu Chemical Co., Ltd.) (note 26: KSG-310, manufactured by Shin-Etsu Chemical Co., Ltd.) (note 27: KF-6038, manufactured by Shin-Etsu Chemical Co., Ltd.) | |

### (Preparation method)

A: Components 4 to 8 and a part of component 3 were uniformly mixed by agitation.
B: Components 9 to 11 and component 13 were mixed.
C: Components 1 to 2 and the rest of component 3 were mixed.
D: B was added into A, and then they were mixed by agitation for emulsification.
E: Component 12 and C were added into D, and then they were mixed by agitation.

### (Result)

The sun-cut cream thus obtained was excellent in adhesion and cosmetic durability with a light and wide spreading property and without greasiness and stickiness.

### [Example 10: sun-cut milky lotion]

| (Components) | (parts by mass) |
|---|---|
| 1. Paste composition obtained in Synthesis Example 7 | 5.0 |
| 2. Paste composition obtained in Synthesis Example 5 | 1.0 |
| 3. Dimethyl polysiloxane (viscosity: 6 mm²/second) | 5.0 |
| 4. Glyceryl trioctanoate | 2.0 |
| 5. Polyether-modified silicone (note 28) | 1.0 |
| 6. Disperse of titanium oxide in decamethyl cyclopentasiloxane (note 29) | 30.0 |
| 7. Disperse of zinc oxide in decamethyl cyclopentasiloxane (note 30) | 30.0 |
| 8. Dipropylene glycol | 3.0 |
| 9. Sodium citrate | 0.2 |
| 10. Preservative | appropriate amount |
| 11. Fragrance | appropriate amount |
| 12. Purified water | 22.8 |

| | |
|---|---|
| (note 28: KF-6017, manufactured by Shin-Etsu Chemical Co., Ltd.) (note 29: SPD-T5, manufactured by Shin-Etsu Chemical Co., Ltd.) (note 30: SPD-Z5, manufactured by Shin-Etsu Chemical Co., Ltd.) | |

### (Preparation method)

A: Components 1 to 5 were uniformly mixed by agitation.
B: Components 8 to 10 and component 12 were mixed.
C: B was added into A, and then they were mixed by agitation for emulsification.
D: Components 6, 7, and 11 were added into C, and then they were mixed by agitation.

### (Result)

The sun-cut milky lotion thus obtained was excellent in cosmetic durability with a light and wide spreading property and without greasiness and stickiness.

As can be seen above, a cosmetic containing the paste composition of the present invention does not have an oily feeling, stickiness, an oil-film feeling, poor spreading properties, or the like, but has a silky and powdery feeling without an oily feeling even if a powder such as a spherical particle is not blended thereinto.

It must be noted here that the present invention is not limited to the embodiments shown above. The embodiments shown above are mere examples so that any embodiments composed of substantially the same technical concept as disclosed in the claims of the present invention and expressing a similar effect thereto are included in the technical scope of the present invention.

## Claims

1. A paste composition comprising at least:
(I) an organopolysiloxane polymer obtained by an addition polymerization of a polysiloxane mixture containing:
(i) one or more organohydrogen polysiloxane shown by the following general formula (1) 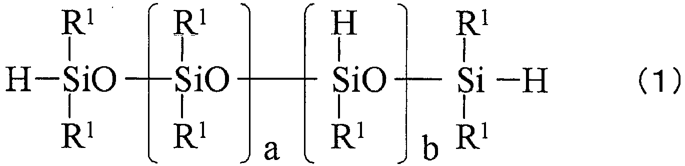 wherein each R¹ may be the same or different and represents a monovalent hydrocarbon group having 1 to 8 carbon atoms and not containing an aliphatic unsaturated group, wherein each of the "a" and "b" represents an integer satisfying a≥1 and b≥0; and
(ii) one or more organopolysiloxane having an aliphatic unsaturated group and shown by the following general formula (3); 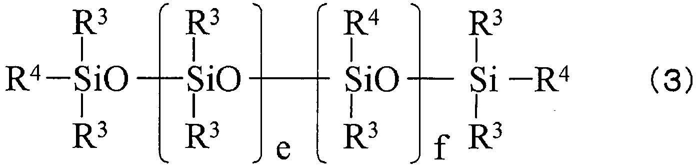 wherein each R³ may be the same or different and represents a monovalent hydrocarbon group having 1 to 8 carbon atoms and not containing an aliphatic unsaturated group, and each R⁴ may be the same or different and represents a monovalent hydrocarbon group having 2 to 8 carbon atoms and containing an aliphatic unsaturated group at its terminal, wherein each of the "e" and "f" represents an integer satisfying e≥1 and f≥0;
wherein 60% or more by mass relative to the total mass of the polysiloxanes shown by the general formulae (1) and (3) is at least two kinds of the polysiloxanes satisfying any of (a+b)≥30 and (e+f)≥30, with the amounts thereof, and
(II) at least one oil material that is a liquid at 25°C, selected from a silicone oil, a hydrocarbon oil, an ester oil, a natural plant and animal oil, and a semi-synthetic oil.

2. The paste composition according to claim 1, wherein the organopolysiloxane polymer obtained by an addition polymerization of a polysiloxane mixture further containing:
one or more organohydrogen polysiloxane shown by the following general formula (2); 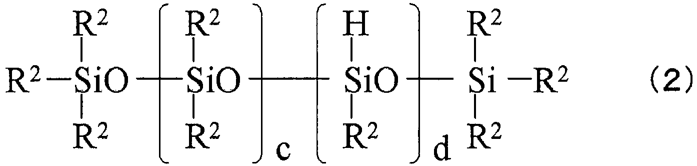 wherein each R² may be the same or different and represents a monovalent hydrocarbon group having 1 to 8 carbon atoms and not containing an aliphatic unsaturated group, wherein each of the "c" and "d" represents an integer satisfying c≥0 and d≥1;
wherein 60% or more by mass relative to the total mass of the polysiloxanes shown by the general formulae (1) to (3) is at least two kinds of the polysiloxanes satisfying any of (a+b)≥30, (c+d)≥30, and (e+f)≥30, with the amounts thereof.

3. The paste composition according to claim 1 or 2, wherein R⁴ in the general formula (3) is a vinyl group.

4. The paste composition according to any one of claims 1 to 3, wherein the oil material is contained therein with the mass ratio thereof to the organopolysiloxane polymer being 1/20 to 20/1.

5. A cosmetic, wherein the paste composition according to any one of claims 1 to 4 is contained therein.

## Patentansprüche

1. Pastenzusammensetzung, umfassend mindestens:
(I) ein Organopolysiloxan-Polymer, erhalten durch Additionspolymerisierung einer Polysiloxan-Mischung, enthaltend:
(i) ein oder mehrere Organowasserstoff-Polysiloxane, wie in der folgenden allgemeinen Formel (1) gezeigt 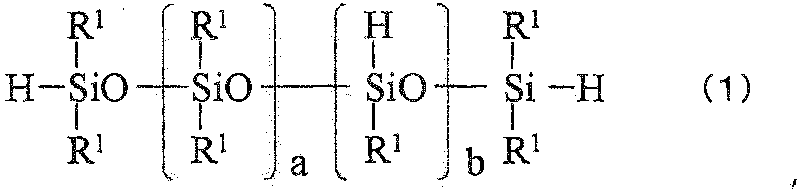 worin jedes R¹ gleich oder verschieden sein kann und monovalente Kohlenwasserstoffgruppe mit 1 bis 8 Kohlenstoffatomen darstellt und nicht eine aliphatische ungesättigte Gruppe enthält, wobei jedes von "a" und "b" für eine Ganzzahl steht, die a≥ und b≥0 genügt; und
(ii) ein oder mehrere Organopolysiloxane mit einer aliphatischen ungesättigten Gruppe und wie in der folgenden allgemeinen Formel (3) gezeigt 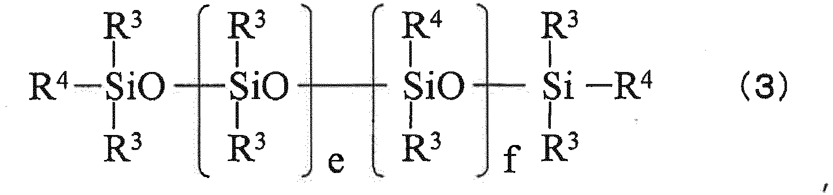 worin jedes R³ gleich oder verschieden sein kann und eine monovalente Kohlenwasserstoffgruppe mit 1 bis 8 Kohlenstoffatomen darstellt und nicht eine aliphatische ungesättigte Gruppe ist, und jedes R⁴ kann gleich oder verschieden sein und eine monovalente Kohlenwasserstoffgruppe mit 2 bis 8 Kohlenstoffatomen darstellen und enthält eine aliphatische ungesättigte Gruppe an seinem Ende, wobei jedes von "e" und "f" für eine Ganzzahl steht, die e≥ und f≥0 genügt;
wobei 60% oder mehr der Masse relativ zur Gesamtmasse der Polysiloxane, wiedergegeben durch die allgemeinen Formeln (1) und (3), mindestens zwei Arten der Polysiloxane ist, genügend einem beliebigen aus (a+b)≥30 und (e+f)≥30, mit den Mengen davon, und
(II) mindestens ein Öl, das bei 25°C flüssig ist, ausgewählt aus einem Silikonöl, einem Kohlenwasserstofföl, einem Esteröl, einem natürlichen Pflanzen- und Tieröl und einem semisynthetischen Öl.

2. Pastenzusammensetzung nach Anspruch 1, worin das durch Additionspolymerisierung einer Polysiloxan-Mischung erhaltene Organopolysiloxan-Polymer weiterhin enthält:
ein oder mehrere Organowasserstoff-Polysiloxane, wie in der folgenden allgemeinen Formel (2) gezeigt 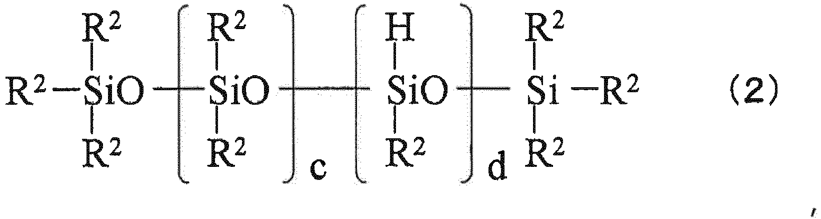
worin jedes R² gleich oder verschieden sein kann und eine monovalente Kohlenwasserstoffgruppe mit 1 bis 8 Kohlenstoffatomen darstellt und nicht eine aliphatische ungesättigte Gruppe enthält, wobei jedes von "c" und "d" für eine Ganzzahl steht, die c≥ und d≥0 genügt;
wobei 60% oder mehr der Masse relativ zur Gesamtmasse der Polysiloxane, wiedergegeben durch die allgemeinen Formeln (1) und (3), mindestens zwei Arten der Polysiloxane ist, genügend einem beliebigen aus (a+b)≥30, (c+d)≥30 und (e+f)≥30, mit den Mengen davon.

3. Pastenzusammensetzung gemäß Anspruch 1 oder 2, worin R⁴ in der allgemeinen Formel (3) eine Vinylgruppe ist.

4. Pastenzusammensetzung nach irgendeinem der Ansprüche 1 bis 3, worin das Ölmaterial darin mit einem Massenverhältnis zum Organopolysiloxan-Polymer von 1:20 bis 20:1 enthalten ist.

5. Kosmetikum, worin die Pastenzusammensetzung gemäß irgendeinem der Ansprüche 1 bis 4 enthalten ist.

## Revendications

1. Composition de pâte comprenant au moins :
(I) un polymère d'organopolysiloxane obtenu par une polymérisation par addition d'un mélange de polysiloxanes contenant :
(i) un ou plusieurs polysiloxanes organohydrogénés présentés par la formule générale (1) suivante 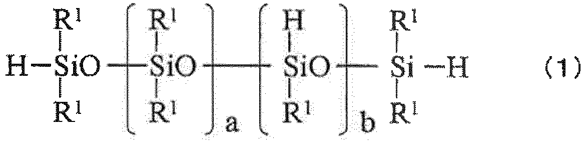 dans laquelle chaque R¹ peut être identique ou différent et représente un groupe hydrocarboné monovalent ayant 1 à 8 atomes de carbone et ne contenant pas de groupe aliphatique insaturé, dans laquelle chacun de « a » et « b » représente un nombre entier satisfaisant a ≥ 1 et b ≥ 0 ; et
(ii) un ou plusieurs organopolysiloxanes ayant un groupe aliphatique insaturé et présentés par la formule générale (3) suivante ; 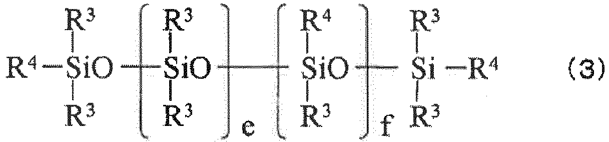 dans laquelle chaque R³ peut être identique ou différent et représente un groupe hydrocarboné monovalent ayant 1 à 8 atomes de carbone et ne contenant pas de groupe aliphatique insaturé, et chaque R⁴ peut être identique ou différent et représente un groupe hydrocarboné monovalent ayant 2 à 8 atomes de carbone et contenant un groupe aliphatique insaturé à son extrémité, dans laquelle chacun de « e » et « f » représente un nombre entier satisfaisant e ≥ 1 et f ≥ 0 ;
dans laquelle 60 % ou plus en masse par rapport à la masse totale des polysiloxanes présentés par les formules générales (1) et (3) sont au moins deux types des polysiloxanes satisfaisant l'une quelconque de (a + b) ≥ 30 et (e + f) ≥ 30, avec les quantités de ceux-ci, et
(II) au moins une matière huileuse qui est liquide à 25°C, choisie parmi une huile de silicone, une huile hydrocarbonée, une huile d'ester, une huile naturelle végétale et animale, et une huile semi-synthétique.

2. Composition de pâte selon la revendication 1, dans laquelle le polymère d'organopolysiloxane obtenu par une polymérisation par addition d'un mélange de polysiloxanes contenant en outre :
un ou plusieurs polysiloxanes organohydrogénés présentés par la formule générale (2) suivante ; 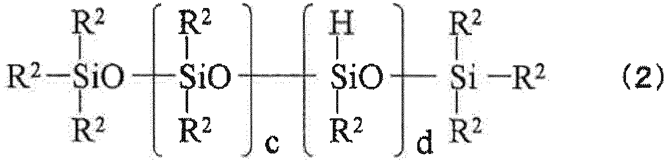
dans laquelle chaque R² peut être identique ou différent et représente un groupe hydrocarboné monovalent ayant 1 à 8 atomes de carbone et ne contenant pas de groupe aliphatique insaturé, dans laquelle chacun de « c » et « d » représente un nombre entier satisfaisant c ≥ 0 et d ≥ 1 ;
dans laquelle 60 % ou plus en masse par rapport à la masse totale des polysiloxanes présentés par les formules générales (1) à (3) sont au moins deux types des polysiloxanes satisfaisant l'une quelconque de (a + b) ≥ 30, (c + d) ≥ 30, et (e + f) ≥ 30, avec les quantités de ceux-ci.

3. Composition de pâte selon la revendication 1 ou 2, dans laquelle R⁴ dans la formule générale (3) est un groupe vinyle.

4. Composition de pâte selon l'une quelconque des revendication 1 à 3, dans laquelle la matière huileuse est contenue dans celle-ci avec le rapport en masse de celle-ci au polymère d'organopolysiloxane étant de 1/20 à 20/1.

5. Produit cosmétique, dans lequel la composition de pâte selon l'une quelconque des revendication 1 à 4 est contenue dans celui-ci.
